# EUROPÄISCHE PATENTSCHRIFT

(11) **EP 2 093 215 B1**
(45) Veröffentlichungstag und Bekanntmachung des Hinweises auf die Patenterteilung: **22.04.2015**
(21) Anmeldenummer: 09001799.7
(22) Anmeldetag: 10.02.2009
(51) Int. Cl.: C07C 263/10, C07C 265/14, C07C 265/12, C07C 263/20, C01B 7/03

(54) **Verfahren zur Herstellung von Isocyanaten**
Method for making isocyanates
Procédé destiné à la fabrication d'isocyanates

(30) Priorität: 19.02.2008 DE 102008009761
(43) Veröffentlichungstag der Anmeldung: 26.08.2009
(73) Patentinhaber: Bayer Intellectual Property GmbH, 40789 Monheim (DE)
(72) Erfinder: Lorenz, Wolfgang, Dr., 41540 Dormagen (DE); Böhm, Matthias, 51373 Leverkusen (DE); Adamson, Richard, 42799 Leichlingen (DE); Steffens, Friedhelm, 51373 Leverkusen (DE); Hielscher, Anke, Houston, TX 77058 (US)
(74) Vertreter: BIP Patents

(56) Entgegenhaltungen:
- EP-A1- 1 849 767
- DE-A1- 10 260 084
- US-A- 3 226 410

## Beschreibung

Die Erfindung betrifft ein Verfahren zur Herstellung von Isocyanaten durch Umsetzung der entsprechenden Amine mit Phosgen, Kondensation des dabei anfallenden Gasgemisches, Strippen der dabei erhaltenen flüssigen Phase und Rückführung des so flüssig zurückerhaltenen Lösungsmittels in die Umsetzung von Amin mit Phosgen. Die gasförmigen Bestandteile werden anschließend in einer Absorption weiter aufgereinigt.

Die Herstellung von Isocyanaten ist seit längerem aus dem Stand der Technik hinlänglich bekannt, wobei in der Regel Phosgen in einem stöchiometrischen Überschuss, bezogen auf das Amin oder einem Gemisch aus zwei oder mehreren Aminen, eingesetzt wird. Verfahren zur Herstellung von organischen Isocyanaten aus primären Aminen und Phosgen sind in der Literatur beschrieben, zum Beispiel in Ullmann's Encyclopedia of Industrial Chemistry, 5th ed. Vol. A 19 p. 390 ff., VCH Verlagsgesellschaft mbH, Weinheim, 1991 und G. Oertel (Ed.) Polyurethane Handbook, 2nd Edition, Hanser Verlag, München, 1993, p. 60 ff. sowie G. Wegener et. Al. Applied Catalysis A: General 221 (2001), p. 303 - 335, Elsevier Science B.V.

Die Synthese des bei der Aminphosgenierung eingesetzten Phosgens ist hinlänglich bekannt und ist z.B. in Ullmann's Enzyklopädie der industriellen Chemie, 3. Auflage, Band 13, Seite 494-500 dargestellt. Weitere Verfahren zur Herstellung von Phosgen sind z.B. in US-A-4764308 und WO-A-03/072237 beschrieben. Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 250°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO-A-03/072237 beschrieben, oder durch Siedekühlung über einen Sekundärkühlkreislauf unter gleichzeitiger Nutzung der Reaktionswärme zur Dampferzeugung, wie z.B. in US-A-4764308 offenbart.

Bei der Aminphosgenierung fällt nicht umgesetztes Phosgen meist zumindest teilweise gasförmig zusammen mit dem freigesetzten Chlorwasserstoff an. Im Verlauf der Isocyanataufarbeitung werden im flüssigen isocyanatführenden Produktstrom noch enthaltene Phosgen- und Chlorwasserstoffanteile abgetrennt. In der Regel können darin noch Anteile an Lösungsmittel, Inertgasen, wie zum Beispiel Stickstoff und Kohlenmonoxid, Nebenprodukte der Phosgensynthese wie zum Beispiel Kohlendioxid und ggf. mitgerissenes Isocyanat enthalten sein. Um das Verfahren zur Herstellung von Isocyanaten möglichst wirtschaftlich zu betreiben, ist es unabdingbar, das überschüssige Phosgen mit möglichst geringen Verlusten zurück zu gewinnen und dem Phosgenierprozess in einer möglichst für das Verfahren optimalen Konzentration im jeweiligen Lösungsmittel wieder zuzuführen sowie das stöchiometrisch anfallende Chlorwasserstoffgas abzutrennen und einer geeigneten Verwertung zu unterziehen, wobei die jeweiligen Verwendungen unterschiedliche Reinheitsanforderungen an den Chlorwasserstoff stellen.

Einsatzmöglichkeiten für Chlorwasserstoff sind zum Beispiel die Vermarktung der wässrigen Lösung (Salzsäure) oder Einsatz von Salzsäure in anderen industriellen oder chemischen Verfahren. Eine der gängigsten Nutzungsmöglichkeiten von gasförmigem Chlorwasserstoff ist die Oxichlorierung von Ethylen mit Chlorwasserstoff zu Ethylendichlorid. Recyclingverfahren für den Chlorwasserstoff und die Rückführung des Chlors und/oder des Wasserstoffs in den Produktionsprozess, in dem der Chlorwasserstoff anfällt zählen ebenfalls zu den bevorzugten Verfahrensweisen. Zu diesen Recyclingverfahren zählen die katalytische Oxidation von Chlorwasserstoff, z.B. nach dem Deacon-Verfahren, die Elektrolyse von gasförmigem Chlorwasserstoff sowie die Elektrolyse einer wässrigen Lösung von Chlorwasserstoff (Salzsäure). Aus WO-A-04/14845 ist ein Verfahren zur katalytischen Oxidation nach dem Deacon-Prozess und aus WO-A-97/24320 ein Verfahren zur Gasphasenelektrolyse von Chlorwasserstoff bekannt. Eine Übersicht über elektrochemische Recycling-Verfahren wird in dem Artikel "Chlorine Regeneration from Anhydrous Hydrogen" von Dennie Turin Mah, veröffentlicht in "12th International Forum Electrolysis in Chemical Industry - Clean and Efficient Processing Electrochemical Technology for Synthesis, Separation, Recycle and Environmental Improvement, October 11-15, 1998, Sheraton Sand Key, Clearwater Beach, FL", gegeben.

Die elektrochemische Oxidation einer wässrigen Lösung von Chlorwasserstoff (Salzsäure) unter Verwendung einer Gasdiffusionselektrode als Kathode ist in WO-A-00/73538 und WO-A-02/18675 beschrieben.

Bei der Elektrolyse von wässrigem Chlorwasserstoff nach dem Diaphragma- oder dem Membranverfahren wird die Salzsäure als Elektrolyt sowohl im Anodenraum als auch im Kathodenraum eingesetzt. Bei der Elektrolyse wird an der Anode Chlor, an der Kathode Wasserstoff erzeugt.

Die genannten Verwertungsmöglichkeiten für Chlorwasserstoff stellen bestimmte Reinheitsanforderungen und bestimmen somit den Aufwand der Reinigung nach Abtrennung des Großteils der anderen Komponenten im Gasstrom Phosgen/Chlorwasserstoff. Die katalytische Chlorwasserstoff-Oxidation nach dem Deacon Prozess wird mit einem Katalysator betrieben, der die Vorreinigung des Chlorwasserstoffgases aus einem Phosgenierprozess mittels Absorption an einem Reinigungsbett beziehungsweise die katalytische Verbrennung von Lösemittelresten im Chlorwasserstoff erfordert (WO-A-04/014845). Bei der Gasphasenelektrolyse von Chlorwasserstoff mit so genannten Festelektrolytsystemen gemäß WO-A-97/24320 ist eine Kontamination der Ionenaustauschermembran oder des katalytisch aktiven Materials unzulässig um ein Austausch der Einheiten zu vermeiden. Bei der elektrochemischen Oxidation einer wässrigen Lösung von Chlorwasserstoff unter Verwendung einer Gasdiffusionselektrode als Kathode wird in WO-A-02/18675 eine Reinigung der Salzsäure mittels Aktivkohle und ggf zusätzlich mittels eines Austauscherharzes vorgeschlagen. Für den Einsatz von Chlorwasserstoffgas in der Oxichlorierung kann zur Abtrennung von störenden Verunreinigungen wie zum Beispiel Lösemittelresten eine zweistufige Kondensation angewandt werden (US-A-6719957).

Eine wässrige Lösung von Chlorwasserstoff (Salzsäure) zum Beispiel für den Einsatz im Nahrungsmittelbereich erfordert eine entsprechend hohe Reinheit, beispielsweise erreichbar durch eine aus dem Stand der Technik bekannte adsorptive Nachreinigung an einem Aktivkohlebett.

Die Behandlung der phosgen- und chlorwasserstoffhaltigen Stoffströme aus der Isocyanatherstellung gemäß dem Stand der Technik wird nachfolgend beschrieben.

Generelles Ziel ist, die Stoffströme Phosgen und Chlorwasserstoff mit darin enthaltenen Nebenkomponenten wie z.B. Lösemittel möglichst ökonomisch in erforderlicher Reinheit zu isolieren, in der Regel um Phosgen beziehungsweise eine Phosgenlösung gewünschter Konzentration in der Aminphosgenierung wieder einzusetzen und Chlorwasserstoff einer geeigneten Verwertung zuführen zu können. Dazu werden üblicherweise die Verfahren der Kondensation, Teilkondensation, Wäsche, Absorption, Adsorption und Destillation angewandt, wie zum Beispiel in EP 1849767 A1 beschrieben.

Eine Partialkondensation von Phosgen aus dem Prozessgas kann unter erhöhtem Druck, zum Beispiel zwischen 10 und 50 bar energetisch günstig mittels Kühlwasser erreicht werden. Ebenfalls wird berichtet, dass sich die Löslichkeit des Phosgens im Lösungsmittel dadurch erhöht, was zu einer Reaktionsbeschleunigung führt. Jedoch sind bei der Durchführung im industriellen Maßstab die erhöhten Sicherheitsvorkehrungen im Hinblick auf eine Leckage mit Phosgenaustritt zu berücksichtigen, wie in DE-A-3212510 beschrieben ist.

Phosgenierreaktion und Aufarbeitung der Gasphase unter erhöhtem Druck wird auch in US-A-3544611 beschrieben. Im Bereich von 10 bis 50 bar wird das Prozessgas mit Wasser gekühlt um einen Grossteil des stöchiometrisch im Überschuss eingesetzten Phosgens zu kondensieren. Eine weitere Phosgenabreicherung im Chlorwasserstoffstrom erfordert den Einsatz von Kältemittel. Hier liegt der ökonomische Vorteil der Aminphosgenierung mit Prozessgasaufarbeitung bei erhöhtem Druck ebenfalls in der Einsparung von Kälteenergie zur Phosgenkondensation und ermöglicht das Arbeiten in konzentrierteren Lösungen, was ebenfalls eine Energieeinsparung darstellt. Alternativ wird in US-A-3544611 eine Ausführungsform beschrieben, bei der Chlorwasserstoff aus dem Prozessgasstrom bei 33 bar und -20°C Kältemitteltemperatur kondensiert wird. In einer Vorstufe wird dabei Phosgen mit Wasserkühlung kondensiert und abgetrennt. Die jeweils geforderte Reinheit der beiden Komponenten wird durch eine Destillations/Strippkolonne zwischen den beiden Kondensationsstufen erreicht. Ein erfinderischer Vorteil der vorliegenden Veröffentlichung gegenüber US-A-3544611 ist die Erzeugung eines gereinigten Lösungsmittelstroms im Verfahrensschritt der Phosgen- und Chlorwasserstofftrennung und Reinigung, der direkt zur Herstellung einer Phosgen- oder auch Aminlösung genutzt werden kann.

In DE-A-10260084 wird auf US-A-3544611 im Hinblick auf ein erhöhtes Gefährdungspotential bei einer Leckage durch diese Druckfahrweise verwiesen. Ferner wird angemerkt, dass sich in den beschriebenen Verfahren eine unerwünscht hohe Chlorwasserstoffkonzentration im Phosgen zur Phosgenierung einstellt, ebenso noch Phosgen mit dem Chlorwasserstoffstrom verloren geht (erste Variante). Bei der zweiten Variante wird neben dem Hinweis auf das bereits genannte Gefährdungspotential auf die energetisch ungünstige Chlorwasserstoffverflüssigung bei tiefen Temperaturen und hohen Drücken verwiesen. Zur Weiterverwertung muss danach der Chlorwasserstoff in der Regel wieder unter Energieeinsatz verdampft werden.

In GB-A-827376 wird eine Aminphosgenierung bei ca. 3 bar durchgeführt. Nach abgeschlossener Reaktion werden in einer Kolonne überschüssiges Phosgen und entstandener Chlorwasserstoff bei erhöhter Temperatur über Kopf abgetrennt. Aus der Gasphase wird Phosgen auskondensiert, der Chlorwasserstoff entspannt und abgeführt. Eine solche einfache Trennung lässt hohe Phosgenrestmengen im Chlorwasserstoff wie auch unerwünscht hohe Chlorwasserstoffgehalte im zurück gewonnenen Phosgen und damit auch in der Phosgenlösung zur Aminphosgenierung erwarten.

Die Aminphosgenierung zu TDI und MDI in Chlorbenzol wird in US-A-3381025 beschrieben. Nach abgeschlossener Reaktion werden Lösungsmittel mit Phosgen und Chlorwasserstoff abdestilliert, anschließend Chlorbenzol und Phosgen kondensiert und der Phosgenierung wieder zugeführt, Chlorwasserstoff mit nicht unerheblichen Restmengen an Phosgen über einen Absorber zur Phosgenvernichtung geführt. Auch hier ist in beiden Strömen die Phosgen-Chlorwasserstoff Auftrennung unvollständig, so dass Phosgenverluste über Chlorwasserstoff erfolgen und unerwünscht hohe Chlorwasserstoffanteile im Phosgen und letztendlich in der Phosgenlösung enthalten sind, die in der Aminphosgenierung nachteilige Aminhydrochloridbildung fördern.

Die in SU-A-1811161 veröffentlichte Aminphosgenierung lehrt, dass der phosgen- und chlorwasserstoffhaltige Prozessgasstrom, abgetrennt vom flüssigen Lösungsmittel-Isocyanatproduktstrom nach mehreren Kondensations- und Absorptionsschritten noch 4% Phosgen in Chlorwasserstoffgas enthält und zu einer weiteren Reinigung und Verwertung abgeleitet wird. Dieser Gasstrom wird vereinigt mit dem Abgas eines Phosgenabsorbers, in dem Gasströme aus der Phosgenherstellung und nicht weiter kondensierbare Ströme aus der Phosgenierung behandelt werden. Dieser Abgasstrom aus dem Absorber wird mit einen Anteil von 4% Chlor beschrieben. Die in diesen Teilströmen genannten Verunreinigungen an Chlor und Phosgen lassen auf einen unvorteilhaften Phosgenherstellungsprozess und entsprechend einer unvollständigen Phosgen-Chlorwasserstofftrennung auf einen verbesserungsfähigen Phosgenierprozess schließen. Ein Verfahrensschritt zur Minimierung des im Umlauf befindlichen Lösungsmittels (Chlorbenzol), analog zur vorliegenden Veröffentlichung, wird nicht beschrieben.

In EP-A-0570799, eine Veröffentlichung zur Aminphosgenierung in der Gasphase, wird auf die Abtrennung von überschüssigem Phosgen nach Kondensation des hergestellten Isocyanats in an sich bekannter Weise verwiesen. Dies kann mittels einer Kühlfalle, Absorption in einem bei einer Temperatur von -10°C bis 8°C gehaltenen inerten Lösemittel (z.B. Chlorbenzol oder Dichlorbenzol) oder durch Adsorption und Hydrolyse an Aktivkohle erfolgen. Letztere Variante erscheint in großtechnischer Ausführung ökonomisch uninteressant. Das die Phosgenrückgewinnungsstufe durchlaufende Chlorwasserstoffgas kann in an sich bekannter Weise zur Rückgewinnung des zur Phosgensynthese erforderlichen Chlors recyclysiert werden.

In US-A-3226410 wird ein kontinuierliches zweistufiges Aminphosgenierverfahren in der Flüssigphase beschrieben. Eine Phosgenlösung wird im stöchiometrischen Überschuss einer Aminlösung im Rohrreaktor bei Temperaturen bis 90°C beigemischt. Die zweite Stufe findet in einem Kessel bei 110 bis 135°C statt. Aus der zweiten Stufe wird die Gasphase, bestehend aus Phosgen, Chlorwasserstoff und Lösemittelanteilen über Kopf entnommen, zweistufig kondensiert und dem Phosgenlösungsbehälter zugeführt. Nichtkondensierbare Anteile gelangen in eine Absorptionskolonne, wo mittels abdestilliertem Lösungsmittel aus der Flüssigphase der Phosgenierung noch im Gasstrom enthaltenes Phosgen absorbiert und dem Phosgenlösungsbehälter zugeführt wird. Nicht absorbierte Anteile aus der Absorptionskolonne, größtenteils Chlorwasserstoffgas, werden einem mit Wasser betriebenen HCl-Absorber in dem wässrige Salzsäure erzeugt wird, zugeführt. In US-A-3226410 wird im Gegensatz zur vorliegenden Erfindung im Phosgenlösungsmittelkreislauf bei der Aminphosgenierung kein Verfahrensschritt beschrieben, in dem die Lösungsmittelmenge zur Gesamtphosgenlösungsmenge gesteuert, bzw. optimiert oder minimiert wird.

Eine für die großtechnische Isocyanatherstellung geringfügiger Bedeutung hat im Hinblick auf die Behandlung und Aufarbeitung der Stoffströme Lösungsmittel, Phosgen und Chlorwasserstoff eine chemische Trennung von Chlorwasserstoff und Phosgen wegen des hohen Einsatzes von z.B. Basen, Verlust des Chlorwasserstoffes und hohem Anfall von Nebenprodukten. Beispielsweise in EP-A-1020435 und DE-A-1233854 werden tertiäre Amine als Chlorwasserstofffänger eingesetzt, die in Form des Hydrochlorids als Feststoffe ausfallen. Alkali- beziehungsweise Erdalkalisalze oder -oxide werden zu diesem Zweck in JP-A-09208589 eingesetzt.

Das Ziel, möglichst reinen Chlorwasserstoff und reines Phosgen aus einem Stoffgemisch, wie es üblicherweise bei der Herstellung von Isocyanaten durch Umsetzung von Aminen mit Phosgen anfällt, zu erhalten, wird in DE-A-10260084 verfolgt. Beschrieben wird ein im Prinzip vierstufiges Verfahren, dessen wesentliche Stufen zwei getrennte Kolonnen nebst Zusatzaggregaten erforderlich machen. Das Prozessgas aus der Isocyanatherstellung besteht überwiegend aus Phosgen, Chlorwasserstoff, Lösemittelanteilen sowie Leichtsiedern und Inerte, beispielhaft seien hier Kohlenmonoxid und Kohlendioxid erwähnt. Der erste Verfahrensschritt ist die partielle Kondensation des Prozessgases, die ein- oder mehrstufig erfolgen kann, wobei je nach Anlagendruck zwischen 40°C mittels Kühlwasser und -40°C mit Solekühlung gearbeitet werden kann. Das so erhaltene teilkondensierte Gemisch wird danach zwischen Abtriebsteil und Verstärkerteil in die nachfolgende Destillationskolonne geführt. Im angegebenen Beispiel mit Chlorbenzol als Lösemittel ist diese Kolonne als Glockenbodenkolonne mit 22 Böden im Abtriebsteil und 11 Böden im Verstärkerteil ausgeführt. Die Kolonne dient zur Entfernung von Chlorwasserstoff aus dem Phosgen und ist dazu mit einem Umlaufverdampfer (Robertverdampfer) und einem Rohrbündelwärmetauscher als Kopfkondensator ausgerüstet. Bei 24,5°C Zulauftemperatur, 38°C Sumpftemperatur, -9°C Kopftemperatur und 2,5 bar Kopfdruck beträgt die Rücklauftemperatur des Teilkondensats am Kolonnenkopf -20°C. Unter diesen Bedingungen wird die Sumpfentnahme mit einem Chlorwasserstoffgehalt von 0,01 Gew.-% angegeben, Phosgen wird mit 89 Gew.-% und Chlorbenzol mit 10 Gew.-% angeführt. Dieser Strom wird dem Reaktionsteil der Isocyanatsynthese zugeführt.

Alternativ zu dem genannten Verdampfer in der Destillationskolonne kann die Chlorwasserstoffentfernung auch durch Strippen mit einem Inertgas wie Stickstoff, dem Prozesslösungsmitteldampf, Phosgen oder einem anderen gasförmigen oder zu verdampfenden Stoff aus dem zu behandelnden Prozessabgas-Strom erfolgen.

Der im Kopfkondensator der Destillationskolonne nicht kondensierte Anteil mit 74 Gew.-% Chlorwasserstoff und 26 Gew.-% Phosgen wird mit -20°C in den unteren Bereich einer Absorptionskolonne geleitet, die mit drei Schüssen Maschendrahtringen bestückt ist. Auf den Wäscherkopf wird Chlorbenzol von -25°C aufgegeben, die Lösungswärme des Chlorwasserstoffs in Chlorbenzol wird mit einem bei -30°C betriebenen Zwischenkühler abgeführt. Am Kopf des Wäschers fallen Brüden an, die nach einem Demister einem bei -30°C betriebenen Kopflcondensator zugeführt werden. Hierbei werden Tröpfchen zurückgehalten, die zusammen mit einem geringen Anteil kondensierter Brüden in den Sumpf dieses Absorbers beziehungsweise Wäschers zurückgeführt werden. Der Kolonnenkopf wird bei 2,2 bar und -8°C, der Sumpf bei 6°C betrieben. Der Kopfentnahme nach dem Kondensator wird ein Chlorwasserstoffgehalt von 99,5 Gew.-%, ein Phosgengehalt von 0,1 Gew.-% bei einem Chlorbenzolgehalt von ebenfalls 0,1 Gew.-% zugewiesen. Der Sumpfaustrag weist 19 Gew.-% Phosgen, 78 Gew.-% Chlorbenzol und 3 Gew.-% Chlorwasserstoff auf.

Im Beispiel von DE-A-10260084 wird der gasförmige Kopfaustrag mit einem Aktivkohlefilter nachgereinigt ohne dass Phosgen- oder Chlorbenzolreste GC-analytisch beziehungsweise IRspektroskopisch nachgewiesen werden konnten.

Der Sumpfaustrag des Absorbers mit oben genannten Gehalten an Phosgen und Chlorwasserstoff soll erfindungsgemäß einer Reaktionskolonne, einer Kolonne zur Phosgenabtrennung oder zur Aufarbeitung des Reaktionsgemisches als Rücklauf zugeführt werden. Im letzten Fall wird auf die Möglichkeit, einen Brüdenkondensator zur Rücklauferzeugung einzusparen, hingewiesen.

DE 10260084 A1 beansprucht die Trennung und Reingewinnung von Chlorwasserstoff und Phosgen bzw. Phosgenlösung zum Wiedereinsatz bzw. zur Weiterverwertung ausgehend von einem Stoffgemisch wie es bei einer Aminphosgenierung anfällt. Eine Optimierung bzw. Erhöhung der Phosgenkonzentration in der Phosgenlösung, erreicht durch das in dieser Erfindung beanspruchte Verfahren oder eine vergleichbare Technologie ohne deutliche Erhöhung des Anlagendrucks, wird nicht beschrieben.

Ausgehend von diesem Stand der Technik stellt sich nun die technische Aufgabe, ein Verfahren zur Herstellung von Isocyanaten umfassend die Reinigung des dabei erhaltenen Stroms enthaltend Chlorwasserstoff, Phosgen und Leichtsieder und Inerte bereit zu stellen, mit welchem Lösungsmittel, Phosgen und Chlorwasserstoff einfach und wirtschaftlich mit hohen Reinheiten wieder erhalten werden kann. Gleichzeitig stellt sich die technische Aufgabe, die Konzentration an Phosgen in der Phosgenlösung in Sumpf der Absorptions- und Konzentrationseinheit (Beispiele in EP 1 849 767 A1, im folgenden kurz Absorber genannt) bei gleichem Druck und gleicher Temperatur einfach und wirtschaftlich erhöhen zu können oder die Chlorwasserstoffreinheit des am Kopf des Absorbers erhaltenen Chlorwasserstoffs bei gleich bleibender Konzentration an Phosgen in der Phosgenlösung im Sumpf des Absorbers durch eine erhöhte Aufgabe von Waschflüssigkeit weiter erhöhen zu können.

In WO 2006/029788 A1 wird ein Verfahren zur Trennung mit der erforderlichen Einzelstoffreinheit von Chlorwasserstoff und Phosgen unter Nutzung von ionischen Lösungsmitteln beschrieben. Bei den möglichen Einsparungen im energetischen Bereich, hat diese Methode den Nachteil, dass zum Beispiel in einem Aminphosgenierprozess mehr als ein Lösungsmittel erforderlich sind.

Das erfindungsgemäße Verfahren bietet gegenüber den Verfahren nach dem Stand der Technik die Möglichkeit, durch eine höhere Phosgenkonzentration in dem im Kreislauf des gesamten Verfahrens geführten Lösungsmittel die benötigte Lösungsmittelmenge im entphosgenierten Isocyanatstrom, die in der Aufarbeitung zum lösungsmittelfreien Isocyanat abgetrennt werden muss, zu verringern. Das bietet einen deutlichen energetischen Vorteil. Alternativ kann bei insgesamt gleichbleibender im Kreislauf befindlicher Lösungsmittelmenge die Lösungsmittelmenge zur Reinigung des Chlorwasserstoffes weiter erhöht werden, falls ein entsprechend gewählter Nachfolgeprozess dies erfordert.

Es wurde nun gefunden, dass dies durch das erfindungsgemäße Verfahren erreicht werden kann. Das erfindungsgemäße Verfahren zeichnet sich dadurch aus, dass es eine Reinigung in einem Kolonnenabtriebsteil (desweiteren Stripper genannt) enthält, in dem mittels Energieeintrag im unteren Kolonnenbereich Chlorwasserstoff und Phosgen gasförmig abgetrieben werden (desweiteren strippen genannt) und weitestgehend gereinigtes Lösungsmittel dem Sumpf des Strippers entnommen wird und anschließend dem Aminphosgenierprozess sowohl als Lösungsmittel für Phosgen als auch für Amin wieder zugeführt werden kann. Dem Stripper vorgeschaltet sind eine oder mehrere Kondensationseinheiten in denen der in Schritt a) des erfindungsgemäßen Verfahrens erhaltene gasförmige Strom enthaltend Chlorwasserstoff, Phosgen, Lösungsmittel sowie Leichtsieder und Inerte teilkondensiert wird. Gemäß Schritt b) erfolgt diese Teilkondensation in einem oder mehreren Schritten wobei die daraus entstehende flüssige Phase im Wesentlichen bestehend aus Lösungsmittel, Phosgen und Chlorwasserstoff auf den Kopf einer Strippkolonne geführt wird und der gasförmige Strom im Wesentlichen enthaltend Chlorwasserstoff, Phosgen sowie Lösungsmittel sowie Leichtsieder und Inerte in einen Absorber (wie z.B. in EP 1 849 767 A1 beschrieben) geleitet wird. Die auf die Strippkolonne gemäß Schritt c) aufgegebene flüssige Phase wird in eine am Kolonnensumpf entnommene, von Phosgenrestmengen befreite Lösungsmittelphase und in einen am Kolonnenkopf entnommenen gasförmigen Strom (Gasphase), der im Wesentlichen aus Phosgen, Chlorwasserstoff, Lösungsmittel (in Konzentrationen von maximal 30 Gew.-% bezogen auf das Gewicht des gasförmigen Stroms), Leichtsiedern und Inerten besteht, aufgetrennt. Zum Energieeintrag ist der Sumpf der Strippkolonne bevorzugt mit einem dampfbetriebenen Umlaufverdampfer bestückt. Um die geforderte Stofftrennung zu erreichen, ist die Kolonne bevorzugt mit einer Packung oder Füllkörpern bestückt. In Frage kommen zum Beispiel Packungen (Gewebe oder Blech) oder Füllkörper (Ringe oder Sättel) aus Metall, Keramik oder Kunststoff wie sie in der Destillationstechnik bzw. Absorptionstechnik gemein hin eingesetzt werden.

Die gemäß Schritt c) erhaltene lösungsmittelarme Gasphase aus dem Kopf der Strippkolonne kann nun alleine oder zusammen mit dem in Schritt b) erhaltenen Gasstrom direkt in einen Absorber (wie in EP 1 849 767 A1 beschrieben) in Schritt e) geleitet werden und bevorzugt vorher in einem oder mehreren Schritten durch eine Teilkondensation geführt werden. Als Wärmetauscher eignen sich alle dem Stand der Technik entsprechenden Kondensatoren. Gegebenenfalls kann frisch hergestelltes gasförmiges Phosgen entweder zusammen mit diesem Strom im Absorber kondensiert und gelöst oder in einer getrennten Phosgenkondensation innerhalb der Phosgenherstellung kondensiert und in den Absorbersumpf eingespeist oder separat in den Reaktionsteil geleitet werden. Gegebenenfalls anfallende phosgenhaltige Lösungsmittelströme aus der Phosgenherstellung, wie z.B. Waschflüssigkeiten, können entweder auf den Kopf des Strippers oder in den Sumpf des Absorbers gegeben werden.

Durch den Einsatz der beschriebenen Strippkolonne kann bereits vor der Phosgenabsorption in Schritt e) ein Großteil des Lösungsmittels, das in dem in Schritt a) erhaltenen Strom enthaltend Chlorwasserstoff, Phosgen, Lösungsmittel sowie Leichtsieder und Inerte enthalten ist, in einer solchen hohen Reinheit, die für den Einsatz zur Herstellung der Aminlösung und Phosgenlösung für den Einsatz in der Phosgenierung in Schritt a) ausreicht, energetisch günstig als Sumpfprodukt abgetrennt werden. Dadurch bleibt allerdings nach der Absorption vorgeschalteten partiellen Kondensation mehr Phosgen in der Gasphase und muss durch einen entsprechend erhöhten Lösungsmittelstrom am Kopf des Absorbers absorbiert werden. Allerdings wird das Lösungsmittel, das mit der Gasphase üblicherweise aus der Reaktion erhalten wird, anschließend bevorzugt unterhalb des Kopfs des Absorbers lediglich gasförmig im Gleichstrom in die Absorptionskolonne aufgegeben, wenn man es nicht wie hier beschrieben vorher durch eine Strippkolonne abtrennt. Daher ist dieser Teil des Lösungsmittels, der danach in der am Sumpf der Absorptionskolonne abgezogenen Phosgenlösung enthalten ist, hinsichtlich seiner Wirksamkeit als Absorptionsmittel weniger effektiv als das Lösungsmittel, das am Kopf des Absorbers im Gegenstrom aufgegeben wird. Durch diesen Effekt kann bei gleicher Phosgenkonzentration im Chlorwasserstoffstrom am Kopf der Absorption eine höhere Phosgenkonzentration in der Phosgenlösung erzielt werden. Dadurch minimiert man die Kreislaufströme für das Prozesslösungsmittel im Bereich der Phosgenabsorption und der Lösungsmittelabtrennung vom Isocyanat, und damit den Energieverbrauch in dem gesamten Verfahren. Besonders hervorzuheben ist, dass durch die Strippkolonne dies bereits bei niedrigen Drücken im Brüdensystem der Phosgenierung sehr effektiv gelingt.

Alternativ zu diesem wirtschaftlichen Vorteil ermöglicht die Erfindung bei Einsatz einer gegenüber EP 1 849 767 A1 entsprechend erhöhten Menge an Waschflüssigkeit für den über den Absorberkopf abgegebenen Chlorwasserstoff eine noch höhere Reinheit dieses Gases zu erreichen, was je nach Einsatz dieses Gasstromes vorteilhaft sein kann (z.B. Einsatz im Deacon Prozess oder als Food Grade HCl). In diesem Fall kann zum Beispiel die durch die Strippkolonne reduzierte Lösungsmittelmenge zur Kopfaufgabe auf den Absorber genutzt werden, wobei die ursprüngliche Phosgenlösungskonzentration, bzw. die ursprüngliche Lösungsmittelmenge im Aminphosgenierkreislauf erhalten bleibt.

Die Erfindung betrifft ein Verfahren zur Herstellung von Isocyanaten, umfassend die folgenden Schritte:
a) Umsetzung von wenigstens einem Amin mit Phosgen in Gegenwart eines Lösungsmittels, wobei ein flüssiger Strom enthaltend das entsprechende Isocyanat und ggf. Lösungsmittel und ein gasförmiger Strom enthaltend Chlorwasserstoff, Phosgen, Lösungsmittel sowie Leichtsieder und Inerte erhalten wird,
b) Auftrennung des gasförmigen Stromes enthaltend Chlorwasserstoff, Phosgen, Lösungsmittel sowie Leichtsieder und Inerte durch partielle Kondensation, wobei eine flüssige Phase im Wesentlichen bestehend aus Lösungsmittel, Phosgen und Chlorwasserstoff erhalten und auf den Kopf einer Strippkolonne geführt wird, und wobei ein gasförmiger Strom im Wesentlichen enthaltend Chlorwasserstoff und Phosgen sowie Lösungsmittel in Konzentrationen von maximal 30 Gew.-%, bevorzugt von maximal 10 Gew.-%, bezogen auf das Gewicht des gasförmigen Stroms, sowie Leichtsieder und Inerte erhalten wird,
c) Auftrennung der in Schritt b) auf die Strippkolonne aufgegebenen flüssigen Phase im Wesentlichen bestehend aus Lösungsmittel, Phosgen und Chlorwasserstoff in eine Lösungsmittelphase, die dem Kolonnensumpf flüssig entnommen wird und die eine Phosgenkonzentration von < 0,1 Gew.-%, bevorzugt < 0,01 Gew.-%, besonders bevorzugt < 0,001 Gew.-%, bezogen auf das Gewicht des flüssigen Sumpfstroms aufweist, sowie eine Gasphase, die am Kolonnenkopf entnommen wird und die Phosgen, Chlorwasserstoff, Leichtsieder und Inerte sowie Lösungsmittel in Konzentrationen von < 30 Gew.-%, bevorzugt von < 10 Gew.-%, besonders bevorzugt von < 5 Gew.-%, bezogen auf das Gewicht der Gasphase enthält,
d) Rückführung der in Schritt c) erhaltenen Lösungsmittelphase in die Phosgenierung in Schritt a),
e) Einleitung der in Schritt c) erhaltenen Gasphase in eine Absorption, in der das in der Gasphase enthaltene Phosgen in dem gleichen Lösungsmittel absorbiert wird, das auch in der Phosgenierung in Schritt a) eingesetzt wird, wobei eine Phosgenlösung mit einer Konzentrationen von 20 - 80 Gew.-%, bevorzugt von 50 - 80 Gew.-% Phosgen, bezogen auf das Gewicht der Phosgenlösung, erhalten wird,
f) Rückführung der erhaltenen Phosgenlösung in die Phosgenierung in Schritt a).

Gegebenenfalls kann die in Schritt e) erhaltene Phosgenlösung mit zusätzlichem Phosgen vermischt und die so erhaltene konzentrierte Phosgenlösung in Schritt f) in die Phosgenierung in Schritt a) zurückgeführt werden.

Bevorzugt enthält das erfindungsgemäße Verfahren noch folgenden weiteren Schritt:
g) Zusammenführung der in den Schritten b), c) und e) erhaltenen gasförmigen Ströme, wobei ein Chlorwasserstoff-Strom enthaltend Phosgen in Konzentrationen von maximal 0,5 Gew.-%, bevorzugt von maximal 0,2 Gew.-%, besonders bevorzugt von maximal 0,1 Gew.-%, bezogen auf das Gewicht des Chlorwasserstoff-Stroms erhalten wird.

Die Umsetzung von Amin und Phosgen in Schritt a) erfolgt bevorzugt in der Flüssigphase in Gegenwart eines Lösungsmittels. Unter den Begriff Lösungsmittel werden dabei organische Lösungsmittel wie o-Dichlorbenzol verstanden, nicht aber inerte Gase wie Stickstoff oder bei niedrigen Temperaturen siedende Komponenten wie Chloroform. Die inerten Gase werden nachfolgend als Inerte bezeichnet, die bei niedrigen Temperaturen siedenden Komponenten (z.B. Chloroform) als Leichtsieder.

Wird die Umsetzung von Amin und Phosgen in Schritt a) in der Flüssigphase in Gegenwart eines Lösungsmittels durchgeführt, so wird als Absorptionsmittel für das Phosgen in Schritt e) das gleiche Lösungsmittel eingesetzt, das auch in der Phosgenierung in Schritt a) als Lösungsmittel eingesetzt wird. Der in Schritt f) in der Absorptionskolonne erhaltene vom Lösungsmittel abgereicherte Phosgenstrom enthält dann bevorzugt neben dem Lösungsmittel 50 bis 80 Gew.-% Phosgen, bezogen auf das Gewicht der Phosgenlösung. Dieser flüssige Phosgenstrom (Phosgenlösung) enthält im Wesentlichen Lösungsmittel und Phosgen und kann dann bevorzugt ohne vorherige Desorption von weiterem Phosgen in die Reaktion in Schritt a) zurückgeführt werden (Schritt g). Zum Ausgleich des Phosgenverbrauchs in der Phosgenierung in Schritt a) wird die Phosgenlösung bevorzugt vorher mit weiterem, bevorzugt frischem Phosgen vermischt. Die Zugabe des frischen Phosgens kann aber beispielsweise auch in die Aminlösung erfolgen.

Wird die Umsetzung von Amin und Phosgen in Schritt a) in der Flüssigphase durchgeführt, so wird weiterhin ein flüssiger Strom enthaltend das entsprechende Isocyanat und ggf. Lösungsmittel erhalten. Bevorzugt enthält dieser Strom, insbesondere wenn das Isocyanat TDI (Toluylendiisocyanat) oder MDI (Di- und Polyisocyanate der Diphenylmethanreihe) ist, weniger als 75 Gew.-%, besonders bevorzugt zwischen 20 und 70 Gew.-%, ganz besonders bevorzugt zwischen 40 und 60 Gew.-%, bezogen auf das Gewicht des flüssigen Stroms, an Lösungsmittel. Dies entspricht einer Verringerung des Gehalts an Lösungsmittel im flüssigen Strom von ca. 10 Gew.-% Lösungsmittel, bezogen auf das Gewicht des gesamten flüssigen Stroms gegenüber den üblichen Flüssigphosgenierverfahren des Standes der Technik, wie zum Beispiel in DE-A-19817691 beschrieben.

Letztendlich und bevorzugt erhält man in Schritt g) aus der Kombination der Gasströme aus der Strippkolonne und dem Phosgenabsorber am Kopf des Absorbers einen gereinigten Strom Chlorwasserstoff (Schritt g), enthaltend Phosgen in Konzentrationen von maximal 0,5 Gew.-%, bevorzugt von maximal 0,2 Gew.-%, besonders bevorzugt von maximal 0,1 Gew.-%, bezogen auf das Gewicht des Chlorwasserstoff-Stroms, der einer weiteren Verwendung zugeführt werden kann.

Bevorzugt erfolgt die Auftrennung des gasförmigen Stroms enthaltend Chlorwasserstoff, Phosgen, Lösungsmittel sowie Leichtsieder und Inerte in Schritt b) und den nachfolgenden Schritten dadurch, dass zunächst aus dem Strom enthaltend Chlorwasserstoff, Phosgen, Lösungsmittel sowie Leichtsieder und Inerte partiell Phosgen und Lösungsmittel auskondensiert wird (Schritt b)). Dieser flüssige Strom wird als Zulauf am Kopf einer Strippkolonne aufgegeben (Schritt c)) und in einen vom Phosgen weitgehend abgereinigten flüssigen Lösungsmittelstrom, der am Sumpf der Kolonne als Lösungsmittel für einen erneuten Einsatz im Verfahren ausgeschleust wird, und einen gasförmigen lösungsmittelarmen Strom, der am Kopf der Kolonne abgezogen wird, aufgetrennt. Bevorzugt wird der in Schritt c) erhaltene gasförmige Strom zunächst wieder in die partielle Kondensation zurückgeführt. Der dabei resultierende gasförmige Gesamtstrom aus der partiellen Kondensation enthaltend Chlorwasserstoff, Phosgen, Lösungsmittel sowie Leichtsieder und Inerte wird anschließend in dem Phosgenabsorber, der bevorzugt wenigstens einen isothermen Absorptionsschritt und wenigstens einen adiabaten Absorptionsschritt enthält, aufgetrennt. Alternativ kann der in Schritt c) erhaltene gasförmige Strom auch direkt in den Phosgenabsorber zur Durchführung des Schrittes e) geleitet werden.

Besonders bevorzugt wird der aus der partiellen Kondensation in Schritt b) resultierende Gasstrom, der den Zulauf für die Strippkolonne liefert, nochmals durch eine partielle Kondensation bei bevorzugt tieferer Temperatur als die vorhergehende, in Schritt b) durchgeführte, partielle Kondensation geleitet, bevor der resultierende Gasstrom anschließend in dem Phosgenabsorber, der bevorzugt wenigstens einen isothermen Absorptionsschritt und wenigstens einen adiabaten Absorptionsschritt enthält, aufgetrennt, und der resultierende Flüssigkeitsstrom in den Sumpf der Absorption gegeben wird.

Besonders bevorzugt erfolgt zusätzlich die Aufgabe von phosgenhaltigen Lösungsmittelströmen aus dem Prozess (Waschflüssigkeiten aus der Phosgenherstellung, Ringflüssigkeiten aus dem Vakuumsystem, Kondensate aus der Lösungsmitteldestillation, etc.) auf den Kopf der Strippkolonne, ggf. auch auf den isothermen Absorptionsschritt (im Gegenstrom), oder ggf. in den Sumpf der Absorptionskolonne.

Besonders bevorzugt erfolgt die isotherme Absorption in Schritt e), bei dem das in Schritt a) eingesetzte Lösungsmittel als Absorptionsmittel eingesetzt wird, im Gegenstrom. Besonders bevorzugt erfolgt die adiabate Absorption in Schritt e), bei dem das in Schritt a) eingesetzte Lösungsmittel als Absorptionsmittel eingesetzt wird, ebenfalls im Gegenstrom.

Besonders bevorzugt wird im Anschluss an die adiabate Absorption im Gegenstrom aus dem Chlorwasserstoff-Strom restliches Lösungsmittel auskondensiert. Bevorzugt wird dieses auskondensierte Lösungsmittel dann ggf. nach Vereinigung mit dem in Schritt c) erhaltenen flüssigen Phosgenstrom in die Umsetzung in Schritt a) zurückgeführt.

Als Absorption wird dabei die Aufnahme und Auflösung von Gasen und Dämpfen in Flüssigkeiten verstanden. Es handelt sich dabei um ein thermisches Trennverfahren, bei dem ein Hilfsstoff, das so genannte Wasch-, Lösungs- oder Absorptionsmittel eingesetzt wird. Durch Desorption oder Strippung kann das Absorbat wieder regeniert werden. Die Gasaufnahme im Lösungsmittel (Absorptionsmittel) wird im Allgemeinen durch tiefe Temperaturen und hohe Drücke begünstigt, umgekehrt sind bei der Desorption höhere Temperaturen und niedrigere Drücke anzuwenden. Die Absorption von Gasen in Lösungsmitteln (Absorptionsmitteln) ist ein exothermer Vorgang, d.h. es wird Wärme frei, die aufgrund des schlechteren Wärmeübergangs nur geringfügig auf die Gasphase und hauptsächlich auf die Flüssigphase übertragen wird. Die Folge der Temperaturerhöhung des Lösungsmittels (Absorptionsmittels) ist nach dem Henryschen Gesetz eine Verringerung des Aufnahmevermögens des Lösungsmittels (Absorptionsmittels) für den zu lösenden Stoff. Im technischen Verfahren bedeutet dies zugleich einen höheren Lösungsmittelbedarf bei konstanter Menge an Absorptiv. Erfolgt keine Abführung der Absorptionswärme bzw. erst nach erfolgter Absorption spricht man von adiabater Absorption. Wird die Absorptionswärme gleichmäßig während der Absorption entfernt und die Temperatur des Lösungsmittels dabei im Wesentlichen konstant gehalten spricht man von isothermer Absorption, die in der Praxis aufgrund der besseren Ausnutzung des Lösungsmittels (Absorptionsmittels), wie bereits beschrieben, bevorzugt ist.

Bevorzugt erfolgt die adiabate Absorption in frischem Lösungsmittel (als Absorptionsmittel), das dem in Schritt a) eingesetzten Lösungsmittel entspricht. Besonders vorteilhaft hat sich im erfindungsgemäßen Verfahren gezeigt, den größten Teil des in dem Strom enthaltend Chlorwasserstoff, Phosgen und gegebenenfalls Lösungsmittel, Leichtsieder und Inerte enthaltenen Phosgens, gegebenenfalls nach partieller Kondensation, mittels einer isothermen Gegenstromabsorption in möglichst wenig Lösungsmittel (Absorptionsmittel) zu absorbieren und die noch verbliebenen Restanteile an Phosgen anschließend mittels adiabater Gegenstromabsorption mit frischem Lösungsmittel (Absorptionsmittel) bei nur geringer adiabater Temperaturerhöhung zu entfernen. Die optimale Austrittskonzentration an Phosgen in dem aus der isothermen Absorption erhaltenen Brüdenstrom (enthaltend Chlorwasserstoff, Phosgen und gegebenenfalls Lösungsmittel, Leichtsieder und Inerte) richtet sich dabei nach der Lösungsmittelmenge (Absorptionsmittelmenge) in der adiabaten Absorption und der, im Hinblick auf das Aufnahmevermögen des Lösungsmittels (Absorptionsmittels), tolerierbaren adiabaten Temperaturerhöhung im Ablauf der adiabaten Absorption. Als zweckmäßig haben sich hier adiabate Temperaturerhöhungen von 0,1 bis 20°C, bevorzugt 2 bis 5°C erwiesen.

Dabei werden bevorzugt Gew.-Verhältnisse von Lösungsmittel zu Phosgen am Eintritt in die isotherme Absorption von 0,1: 1 bis 10 : 1, besonders bevorzugt von 1 : 1 bis 3 : 1 eingesetzt. Das eingesetzte Lösungsmittel für die isotherme Absorption kann dabei entweder komplett oder auch teilweise bereits am Kopf der adiabaten Absorption aufgegeben werden.

Die Ausführungsform und die Betriebsbedingungen der Phosgenabsorptionskolonne in Schritt e) können wie in EP 1 849 767 A1 beschrieben gewählt werden, d.h. bevorzugt liegt die Kopftemperatur im Bereich von -40 bis 0°C, besonders bevorzugt von -30 bis -20°C. Der absolute Kopfdruck liegt bevorzugt zwischen 1 und 35 bar, besonders bevorzugt zwischen 1,2 und 3 bar.

Ggf. kann in Schritt f) die in Schritt e) erhaltene Phosgenlösung mit zusätzlichem Phosgen vermischt werden, wobei eine konzentrierte Phosgenlösung erhalten wird. Bevorzugt wird dieser Schritt f) durchgeführt.

Die Rückführung der in Schritt e) erhaltenen Phosgenlösung und / oder der gegebenenfalls erhaltenen konzentrierten Phosgenlösung in die Phosgenierung in Schritt a) kann dabei so ausgestaltet sein, dass die gesamte in Schritt e) erhaltenen Phosgenlösung und / oder die gesamte ggf. erhaltene konzentrierte Phosgenlösung zurückgeführt wird. Es ist jedoch auch möglich, nur einen Teil der in Schritt e) erhaltenen Phosgenlösung und / oder der ggf. erhaltenen konzentrierten Phosgenlösung zurückzuführen. Bevorzugt werden mehr als 10%, besonders bevorzugt mehr als 50 %, ganz besonders bevorzugt zwischen 60 und 100%, insbesondere bevorzugt zwischen 65 und 99,95% der in Schritt e) erhaltenen Phosgenlösung und / oder der ggf. erhaltenen konzentrierten Phosgenlösung in die Phosgenierung in Schritt a) zurückgeführt.

Die Isocyanatherstellung durch die Umsetzung von Amin mit Phosgen in Schritt a), die so genannte Phosgenierung, findet im großtechnischen Maßstab üblicherweise in der Flüssigphase statt, wobei das Phosgen und das Amin in einem Lösungsmittel gelöst sein können. Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. *β*-Naphthylchlorid, Benzoesäureethylester, Phthalsäuredialkylester, Diisodiethylphthalat, Toluol und Xylole sowie Methylenchlorid, Perchlorethylen, Trichlorfluormethan oder Butylacetat. Gemische der beispielhaft genannten Lösemittel können ebenfalls eingesetzt werden. Weitere Beispiele für geeignete Lösemittel sind aus dem Stand der Technik bekannt. Wie außerdem aus dem Stand der Technik (z.B. WO-A-96/16028) bekannt, kann als Lösungsmittel für Phosgen ebenso das gebildete Isocyanat selbst fungieren.

Grundsätzlich kann die Phosgenierung, insbesondere geeigneter aromatischer und aliphatischer Diamine, auch in der Gasphase, d.h. oberhalb des Siedepunktes des Amins, stattfinden. Die Gasphasenphosgenierung ist z.B. in EP-A-570 799 beschrieben. Vorteile dieses Verfahrens gegenüber der ansonsten üblichen Flüssigphasenphosgenierung liegen in der Energieeinsparung, bedingt durch die Minimierung eines aufwändigen Lösungsmittel- und Phosgenkreislaufs. In der Regel wird auch in der Gasphasenphosgenierung Lösemittel eingesetzt, jedoch in geringeren Mengen als in der Flüssigphasenphosgenierung, wie zum Beispiel in DE-A-10245704 beschrieben, so dass auch hier prinzipiell die Trennaufgabe von Phosgen, Chlorwasserstoff und Lösungsmittel besteht.

Als organische Amine eignen sich prinzipiell alle primären Amine mit einer oder mehreren primären Aminogruppen, die mit Phosgen unter Bildung eines oder mehrerer Isocyanate mit einer oder mehreren Isocyanatgruppen reagieren können. Die Amine weisen mindestens eine, bevorzugt zwei, oder gegebenenfalls drei und mehr primäre Aminogruppen auf. So kommen als organische primäre Amine aliphatische, cycloaliphatische, aliphatisch-aromatische, aromatische Amine, Di- und/oder Polyamine in Frage, wie zum Beispiel Methylamin, Ethylamin, Butylamin, Stearylamin, Anilin, Halogen-substituierte Phenylamine, z.B. 4-Chlorphenylamin, 1,4-Diaminobutan, 1,6-Diaminohexan, 1,8-Diaminooctan, 1-Amino-3,3,5-trimethyl-5-amino-cyclohexan, Lysinethylester, Lysinaminoethylester, 1,6,11-Triaminoundecan oder 1,5- und / oder 1,8- Naphthylendiamin, 1,4-Diaminobenzol, p-Xylylendiamin, perhydriertes 2,4- und/oder 2,6-Diaminotoluol, 2,2'-, 2,4'-und/oder 4,4'-Diaminodicyclohexylmethan, 2,4-, 2,6-Diaminotoluol oder deren Gemische, 4,4'-, 2,4'- oder 2,2'-Diphenylmethandiamin oder deren Gemische, wie auch höhermolekulare isomere, oligomere oder polymere Derivate der genannten Amine und Polyamine. Weitere mögliche Amine sind aus dem Stand der Technik bekannt. Bevorzugte Amine für die vorliegende Erfindung sind die Di- und Polyamine der Diphenylmethan-Reihe (MDA, monomere, oligomere und polymere Amine), 2,4-, 2,6-Diaminotoluol (TDA, Toluylendiamine), beispielsweise technische Gemische von 2,4-, 2,6-Diaminotoluol (TDA, Toluylendiamine) im Gewichtsverhältnis 80:20, Isophorondiamin und Hexamethylendiamin. Bei der Phosgenierung erhält man die entsprechenden Isocyanate: Di- und Polyisocyanate der Diphenylmethanreihe (d.h. MDI: monomere, oligomere und polymere Isocyanate), Toluylendiisocyanat (TDI), Hexamethylendüsocyanat (HDI) und Isophorondiisocyanat (IPDI).

Die Amine können mit Phosgen in einer einstufigen oder zweistufigen oder ggf. mehrstufigen Reaktion umgesetzt werden. Dabei ist eine kontinuierliche wie auch diskontinuierliche Betriebsweise möglich.

Wird eine einstufige Phosgenierung in der Gasphase gewählt, so erfolgt die Umsetzung oberhalb der Siedetemperatur des Amins bevorzugt innerhalb einer mittleren Kontaktzeit von 0,5 bis 5 Sekunden und bei Temperaturen von 200 bis 600°C.

Bei der Phosgenierung in der Flüssigphase werden üblicherweise Temperaturen von 20 bis 240°C und Drücke von 1 bis ca 50 bar absolut eingesetzt. Die Phosgenierung in der Flüssigphase kann einstufig oder mehrstufig durchgeführt werden, wobei Phosgen im stöchiometrischen Überschuss eingesetzt werden kann. Dabei werden die Aminlösung und die Phosgenlösung bevorzugt über ein statisches Mischelement vereinigt und anschließend beispielsweise von unten nach oben durch einen oder mehrere Reaktionstürme geführt, wo das Gemisch zum gewünschten Isocyanat ausreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt (EP 0830894 A1, EP 0291820 A1).

In der Regel wird die kontinuierliche Flüssigphasen-Isocyanatherstellung im industriellen Maßstab zweistufig durchgeführt. Dabei wird in der ersten Stufe im Allgemeinen bei Temperaturen von maximal 220°C, bevorzugt maximal 160°C aus Amin und Phosgen das Carbamoylchlorid sowie aus Amin und abgespaltenem Chlorwasserstoff Aminhydrochlorid gebildet. Diese erste Stufe ist stark exotherm. In der zweiten Stufe wird sowohl das Carbamoylchlorid zu Isocyanat und Chlorwasserstoff gespalten als auch das Aminhydrochlorid zum Carbamoylchlorid umgesetzt. Die zweite Stufe wird in der Regel bei Temperaturen von mindestens 90°C, vorzugsweise von 100 bis 240°C, durchgeführt.

Nach dem Verfahrenschritt der Aminphosgenierung erfolgt in der Regel die Abtrennung der bei der Phosgenierung gebildeten Isocyanate. Dies geschieht beispielsweise dadurch, dass zunächst das Reaktionsgemisch der Phosgenierung in einen flüssigen und einen gasförmigen Produktstrom aufgetrennt wird. Hierzu geeignete Verfahren wie Kondensation oder Wäsche sind allgemein bekannt, und beispielsweise in US-A-3544611 und GB-A-827376 beschrieben. Der flüssige Produktstrom enthält im Wesentlichen das Isocyanat bzw. Isocyanatgemisch, ggf. eingesetztes Lösungsmittel sowie einen geringen Teil an nicht umgesetztem Phosgen. Der gasförmige Produktstrom besteht im Wesentlichen aus Chlorwasserstoffgas, überschüssigem Phosgen, dem thermodynamischen Gleichgewicht entsprechenden Mengen an Lösemittel sowie Nebenprodukten aus der Phosgenerzeugung wie zum Beispiel Kohlendioxid. Aus der weiteren Aufarbeitung des flüssigen Produktstromes erfolgt ggf. eine weitere Abtrennung von Restmengen an Phosgen.

Daher ist vor allem aus ökonomischer Sicht eine möglichst vollständige Rückgewinnung überschüssigen Phosgens zum erneuten Einsatz in der Phosgenierung, eine Reduzierung des Lösungsmittelkreislaufs auf ein ökonomisch sinnvolles Minimum sowie eine Isolierung möglichst reinen Chlorwasserstoffs zur Weiterverwertung angestrebt.

Die destillative Abtrennung und Reinigung des Lösungsmittels aus dem flüssigen Produktstrom und dessen Rückführung in die Stufe der Aminphosgenierung kann nach einem der aus dem Stand der Technik bekannten Verfahren, beispielsweise nach dem in DE-A-102006022448 (und darin enthaltenen Zitaten) beschriebenen Verfahren, durchgeführt werden.

Der gasförmige Strom enthaltend Chlorwasserstoff, Phosgen, Lösungsmittel sowie Leichtsieder und Inerte, der bei der Umsetzung von aliphatischen oder aromatischen Aminen mit Phosgen zu den entsprechenden Isocyanaten in Schritt a) in der Flüssigphasenphosgenierung üblicherweise anfällt, enthält bevorzugt 20 bis 75 Gew.-% Phosgen, 5 bis 50 Gew.-% Lösungsmittel und 5 bis 50 Gew.-% Chlorwasserstoff, bezogen auf das Gewicht des Stroms. Die Variationsbreite der Zusammensetzung des Stroms enthaltend Chlorwasserstoff, Phosgen, Lösungsmittel sowie Leichtsieder und Inerte ergibt sich dabei aus der Möglichkeit, unterschiedliche Verfahren der Phosgenierung einzusetzen, welche durch unterschiedliche Drücke, Temperaturen und Prozesslösungsmittel gekennzeichnet sind, oder gegebenenfalls in der Gasphase bzw. lösemittelfrei erfolgen können.

Die Strippung des in Schritt b) gewonnenen Kondensates im Wesentlichen bestehend aus Lösungsmittel, Chlorwasserstoff und Phosgen wird in dem erfindungsgemäßen Verfahren in Schritt c) in einer Strippkolonne ausgeführt. Es handelt sich dabei üblicherweise um eine Kolonne bestehend aus einem Abtriebsteil (d.h. der Zulauf erfolgt am Kopf der Kolonne) und einem Sumpfverdampfer, der üblicherweise als Naturumlauf-, Zwangsumlauf- oder Fallfilmverdampfer ausgeführt ist. Der Zusatz eines zusätzlichen Strippmittels ist möglich, bevorzugt wird aber kein Strippmittel zugegeben sondern das im Sumpf der Strippkolonne verdampfte Phosgen und Chlorwasserstoff dient als Strippmittel. Der Kolonnenkörper hat im technischen Maßstab bevorzugt einen Durchmesser von 0,1 bis 4,0m, bevorzugt 0,5 bis 2,0m. Der mit Stoffaustauscheinbauten versehene Bereich in der Kolonne liegt üblicherweise, je nach Typ, zwischen 1,0 und 10,0m, bevorzugt zwischen 2,0 und 8,0m, besonders bevorzugt zwischen 4,0 und 6,0m.

Als Einbauten zur Bereitstellung einer möglichst hohen Stoffaustauschfläche können alle aus dem Stand der Technik bekannten und dem Fachmann geläufigen Füllkörper, Packungen oder Stoffaustauschböden eingesetzt werden. Dabei kann es sich um gängige Packungstypen wie z.B. B1, C1 (Fa. Montz) oder Mellapak, Kerapak (Fa. Sulzer) oder Ralupak (Fa. Raschig) oder Rombopak (Fa. Kühni) oder auch um alle anderen gängigen Packungstypen für die Rektifikation oder Füllkörpertypen (alle von Fa. VFF bis auf speziell gekennzeichnete) wie z.B. Raschig-Ringe, Pall-Ringe, oder Sättel vom Typ Intalox, Berl, Super-Torus (Fa. Raschig) oder Super, Interpack, Top-Pak, Hacketten, Igel, VSP oder Hiflow-Ring (Fa. Rauschert) in den kommerziell verfügbaren Größen und den für das Stoffsystem bei den herrschenden Bedingungen, dem Fachmann geläufigen, beständigen Werkstoffen handeln. Die Aufgabe des Zulaufs am Kopf der Strippkolonne auf Füllkörper oder Packung kann dabei bevorzugt über einen, dem Stand der Technik entsprechenden Flüssigkeitsverteiler erfolgen. Bei Stoffaustauschböden kann der Zulauf über einen, dem Stand der Technik entsprechenden Zulaufboden aufgegeben werden. Der absolute Kopfdruck der Strippkolonne ergibt sich zwangsweise aus dem Kopfdruck der eingesetzten Absorptionskolonne und den in der Kolonne auftretenden Druckverlusten und liegt damit bevorzugt zwischen 1 und 35 bar, besonders bevorzugt zwischen 1,2 und 3 bar. Die Sumpftemperatur ergibt sich je nach Druckverlust in der Kolonne und eingesetztem Lösungsmittel bevorzugt zu 130 bis 410°C, besonders bevorzugt zu 135 bis 230°C. Der Lösungsmittelstrom (Lösungsmittelphase) aus dem Sumpf des Strippers hat eine Phosgenkonzentration von < 0,1 Gew%, bevorzugt < 0,01 Gew%, besonders bevorzugt < 0,001 Gew%, bezogen auf den flüssigen Lösungsmittelstrom (Lösungsmittelphase).

Die Rückführung der in Schritt c) erhaltenen Lösungsmittelphase in die Phosgenierung in Schritt a), die Gegenstand des Schrittes d) ist, kann dabei so ausgestaltet sein, dass die gesamte in Schritt c) erhaltene Lösungsmittelphase zurückgeführt wird. Es ist jedoch möglich, nur einen Teil der in Schritt c) erhaltenen Lösungsmittelphase zurückzuführen. Bevorzugt werden mehr als 10%, besonders bevorzugt mehr als 50 %, ganz besonders bevorzugt zwischen 60 und 100%, insbesondere bevorzugt zwischen 65 und 99,95% der in Schritt c) erhaltenen Lösungsmittelphase in die Phosgenierung in Schritt a) zurückgeführt.

Die Erfindung wird im Folgenden anhand der Figuren näher erläutert. Es zeigen:

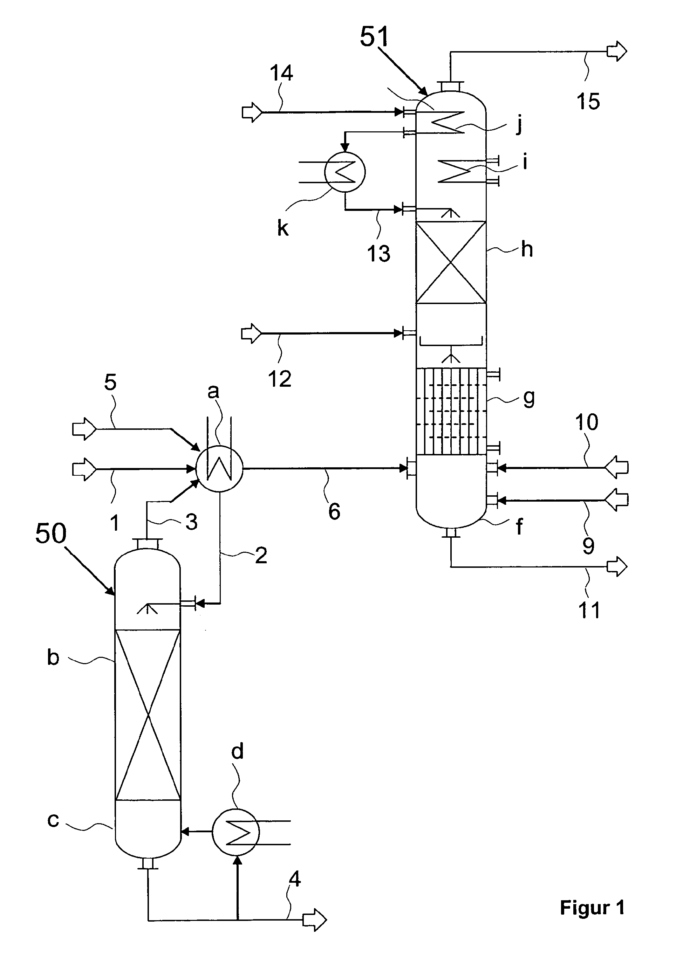

- Figur 1: eine schematische Darstellung der Auftrennung des gasförmigen Stroms enthaltend Chlorwasserstoff, Phosgen, Lösungsmittel sowie Leichtsieder und Inerte in einer Kombination von einer ein- oder mehrstufigen partiellen Kondensation, gefolgt von einer Strippung des erhaltenen Kondensates und weiterer Auftrennung des Brüdenstromes in einer Absorptionskolonne,

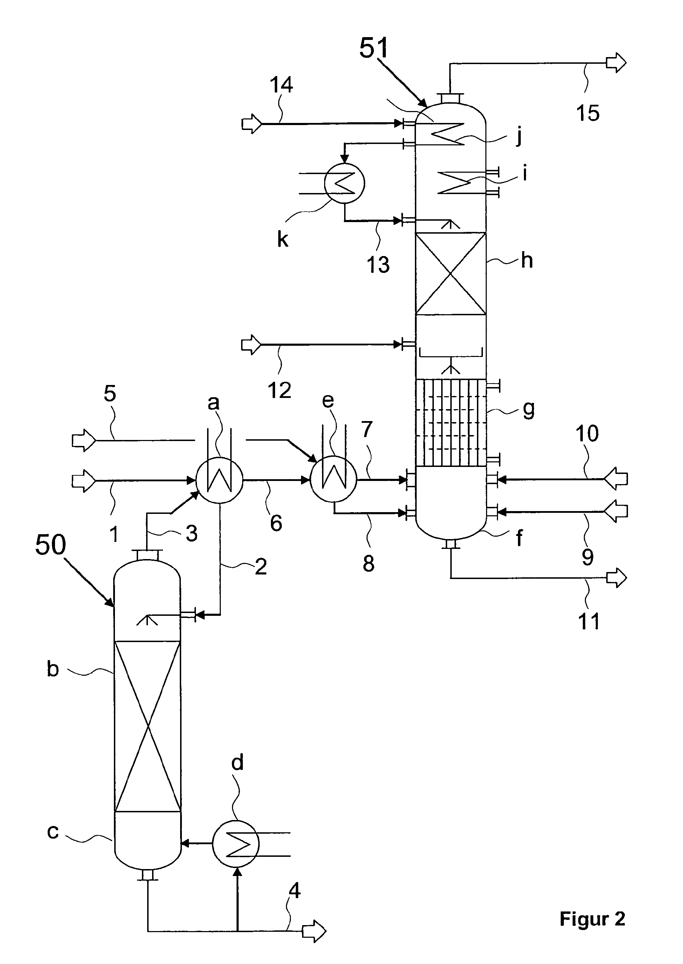

- Figur 2: eine schematische Darstellung der Auftrennung des gasförmigen Stroms enthaltend Chlorwasserstoff, Phosgen, Lösungsmittel sowie Leichtsieder und Inerte in einer Kombination von einer ein- oder mehrstufigen partiellen Kondensation, gefolgt von einer Strippung des erhaltenen Kondensates und nochmaliger ein- oder mehrstufiger partieller Kondensation bei entsprechend tieferer Temperatur als in der partiellen Kondensation vor der Strippkolonne und weiterer Auftrennung des Brüdenstromes in einer Absorptionskolonne,

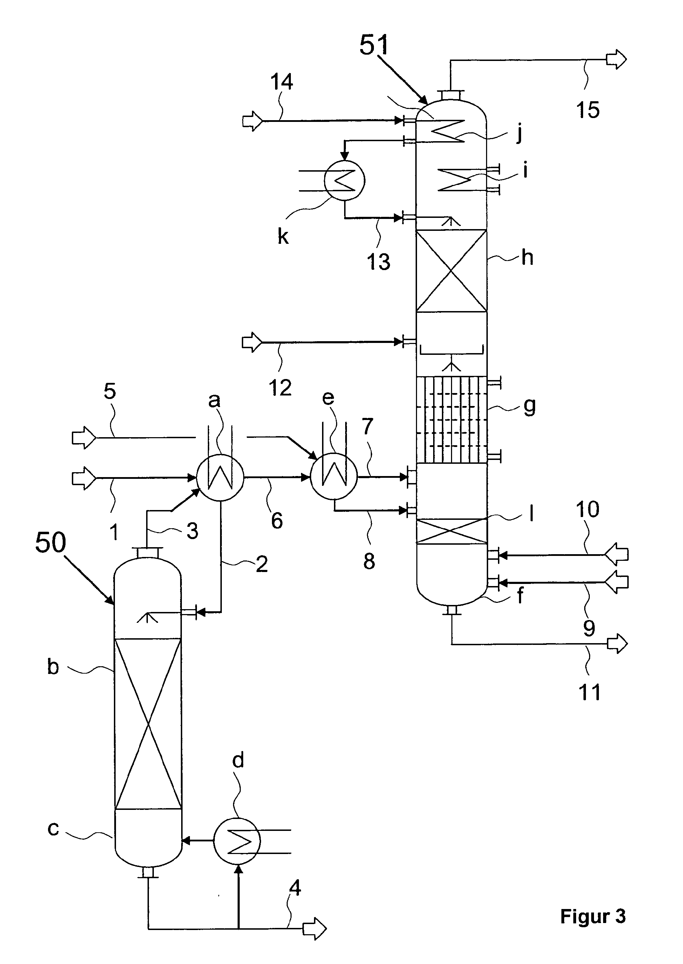

- Figur 3: eine schematische Darstellung der Auftrennung des gasförmigen Stroms enthaltend Chlorwasserstoff, Phosgen, Lösungsmittel sowie Leichtsieder und Inerte in einer Kombination von einer ein- oder mehrstufigen partiellen Kondensation, gefolgt von einer Strippung des erhaltenen Kondensates und nochmaliger ein- oder mehrstufiger partieller Kondensation bei entsprechend tieferer Temperatur als in der partiellen Kondensation vor der Strippkolonne und weiterer Auftrennung des Brüdenstromes in einer Absorptionskolonne sowie Strippung der Phosgenlösung.

Figur 1 zeigt eine schematische Darstellung der Auftrennung des gasförmigen Stroms enthaltend Chlorwasserstoff, Phosgen, Lösungsmittel sowie Leichtsieder und Inerte in einer Kombination von einer ein- oder mehrstufigen partiellen Kondensation (Schritt b)), gefolgt von einer Strippung des erhaltenen Kondensates (Schritt c)) und weiterer Auftrennung des Brüdenstromes in einer Absorptionskolonne (Schritt e)).

Der gasförmige Strom 1 enthaltend Chlorwasserstoff, Phosgen, Lösungsmittel sowie Leichtsieder und Inerte, der üblicherweise bei der Umsetzung von aliphatischen oder aromatischen Aminen mit Phosgen zu den entsprechenden Isocyanaten anfällt, wird in einer bevorzugten Ausführungsform der Erfindung zur Auftrennung zunächst in eine ein- oder mehrstufige partielle Kondensation a geleitet. Zusätzlich können dort phosgenhaltige Lösungsmittelströme 5 aus dem Isocyanatprozess aufgegeben werden, wie sie üblicherweise als Waschflüssigkeiten aus der Phosgenherstellung, Ringflüssigkeiten aus dem Vakuumsystem, Kondensate aus der Lösungsmitteldestillation, etc. anfallen können. Als Wärmetauscher eignen sich alle dem Stand der Technik entsprechenden Kondensatoren. Der Kondensationsschritt erfolgt üblicherweise mit luftgekühlter Kühlsole, bei bevorzugt 25 bis 50°C, besonders bevorzugt bei 30 bis 40°C. Das dabei entstehende Kondensat 2 wird als Zulauf auf den Kopf einer Strippkolonne 50 gegeben. Der Zulauf wird im strukturierten Packungsteil b der Kolonne in einen Kopfbrüdenstrom 3, der weitestgehend von Lösungsmittel abgereichert ist und wieder in die erste partielle Kondensationsstufe a geleitet wird, und in einen von Phosgen weitestgehend befreiten Lösungsmittelstrom 4 am Sumpf c der Kolonne aufgetrennt. Die dazu notwendigen Brüden werden aus der aus der Packung b ablaufenden Flüssigkeit mit dem Sumpfverdampfer d erzeugt.

Der Brüdenstrom 6 aus der ersten partiellen Kondensationsstufe a wird in eine Phosgenabsorptionskolonne 51, einer Kombination aus isothermer g und adiabater Absorption h mit Nachkondensation i des Lösungsmittels und Energierückgewinnung j aus dem nur noch schwach Phosgen bzw. Lösungsmittelhaltigen Chlorwasserstoffstrom, zur weiteren Auftrennung geleitet. Zusätzlich werden kondensiertes Phosgen 9 und ggf. größtenteils durch eine Waschkolonne in Chlorbenzol zurückgewonnenes nichtkondensierbares Restphosgen 10 aus der Phosgenherstellung in den Sumpf f der Absorptionskolonne 51 gegeben. Der Zulauf 12 am Kopf der isothermen Absorption g besteht aus Lösungsmittel das ggf. auch durch Lösungsmittelströme die mit geringen Mengen Phosgen verunreinigt sind (wie z.B. Waschflüssigkeiten aus der Phosgenherstellung, Ringflüssigkeiten aus dem Vakuumsystem, Kondensate aus der Lösungsmitteldestillation, etc.) ersetzt werden kann. Die Kühlung der isothermen Absorption wird bevorzugt mit Kaltsole bei bevorzugt -40 bis +0°C, besonders bevorzugt bei -20 bis -10°C durchgeführt.

Der durch die isotherme Absorption g vorgereinigte Chlorwasserstoffstrom wird intern der adiabaten Absorptionsstufe h zugeführt und dort weiter von Phosgen befreit. Zur Absorption wird im Gegenstrom Lösungsmittel 13, das durch den Wärmetauscher k mit Kaltsole auf Temperaturen von bevorzugt -50 bis +0°C, besonders bevorzugt -40 bis -20°C abgekühlt wurde, als Strom 14 am Kopf der adiabaten Absorption h aufgegeben. Der von Phosgen in der adiabaten Absorption h und von Lösungsmittel in der Nachkondensation i abgereinigte Chlorwasserstoffstrom 15 tritt am Kopf der Absorptionskolonne 51 aus. Die Kühlung der Nachkondensation erfolgt mit Kaltsole von bevorzugt -50 bis +0°C, besonders bevorzugt -40 bis -20°C. Zur Energierückgewinnung wird der Lösungsmittelstrom 13 im Wärmetauscher j vorgekühlt und der Chlorwasserstoffstrom 15 aufgewärmt.

Am Sumpf f der Phosgenabsorptionskolonne wird eine konzentrierte Phosgenlösung 11 abgezogen und zurück in die Reaktionsstufe gefördert.

Figur 2 zeigt eine schematische Darstellung der Auftrennung des gasförmigen Stroms enthaltend Chlorwasserstoff, Phosgen, Lösungsmittel sowie Leichtsieder und Inerte in einer Kombination von einer ein- oder mehrstufigen partiellen Kondensation (Schritt b)), gefolgt von einer Strippung des erhaltenen Kondensates (Schritt c)) und nochmaliger ein- oder mehrstufiger partieller Kondensation bei entsprechend tieferer Temperatur als in der partiellen Kondensation vor der Strippkolonne und weiterer Auftrennung des Brüdenstromes in einer Absorptionskolonne (Schritt e)).

Der gasförmige Strom 1 enthaltend Chlorwasserstoff, Phosgen, Lösungsmittel sowie Leichtsieder und Inerte, der üblicherweise bei der Umsetzung von aliphatischen oder aromatischen Aminen mit Phosgen zu den entsprechenden Isocyanaten anfällt, wird in einer bevorzugten Ausführungsform der Erfindung zur Auftrennung zunächst in eine ein- oder mehrstufige partielle Kondensation a geleitet. Zusätzlich können dort oder in der partiellen Kondensationsstufe e nach der Strippkolonne phosgenhaltige Lösungsmittelströme 5 aus dem Isocyanatprozess aufgegeben werden, wie sie üblicherweise als Waschflüssigkeiten aus der Phosgenherstellung, Ringflüssigkeiten aus dem Vakuumsystem, Kondensate aus der Lösungsmitteldestillation, etc. anfallen können. Als Wärmetauscher eignen sich alle dem Stand der Technik entsprechenden Kondensatoren. Der Kondensationsschritt erfolgt üblicherweise mit luftgekühlter Kühlsole, bei bevorzugt 25 bis 50°C, besonders bevorzugt bei 30 bis 40°C. Das dabei entstehende Kondensat 2 wird als Zulauf auf den Kopf einer Strippkolonne 50 gegeben. Der Zulauf wird im strukturierten Packungsteil b der Kolonne in einen Kopfbrüdenstrom 3, der weitestgehend von Lösungsmittel abgereichert ist und wieder in die erste partielle Kondensationsstufe a geleitet wird, und in einem von Phosgen weitestgehend befreiten Lösungsmittelstrom 4 am Sumpf c der Kolonne aufgetrennt. Die dazu notwendigen Brüden werden aus der Flüssigkeit, die aus der Packung b abläuft, mit dem Sumpfverdampfer d erzeugt.

Der Brüdenstrom 6 aus der ersten partiellen Kondensationsstufe a wird über eine zweite, bei tieferer Temperatur als die Kondensationsstufe a betriebene, ein- oder mehrstufige partielle Kondensation e geleitet. Als Wärmetauscher eignen sich alle dem Stand der Technik entsprechenden Kondensatoren. Der Kondensationsschritt wird bevorzugt mit Kaltsole bei bevorzugt -40 bis +10°C, besonders bevorzugt bei -20 bis -10°C durchgeführt. Die resultierenden Brüden- 7 und Kondensat-Ströme 8 werden in eine Phosgenabsorptionskolonne 51, einer Kombination aus isothermer g und adiabater Absorption h mit Nachkondensation i des Lösungsmittels und Energierückgewinnung j aus dem nur noch schwach Phosgen bzw. Lösungsmittelhaltigen Chlorwasserstoffstrom, zur weiteren Auftrennung geleitet. Zusätzlich werden kondensiertes Phosgen 9 und ggf. größtenteils durch eine Waschkolonne in Chlorbenzol zurückgewonnenes nichtkondensierbares Restphosgen 10 aus der Phosgenherstellung in den Sumpf f der Absorptionskolonne 51 gegeben. Der Zulauf 12 am Kopf der isothermen Absorption g besteht aus Lösungsmittel das ggf. auch durch Lösungsmittelströme die mit geringen Mengen Phosgen verunreinigt sind (wie z.B. Waschflüssigkeiten aus der Phosgenherstellung, Ringflüssigkeiten aus dem Vakuumsystem, Kondensate aus der Lösungsmitteldestillation, etc.) ersetzt werden kann. Die Kühlung der isothermen Absorption wird bevorzugt mit Kaltsole bei bevorzugt -40 bis +0°C, besonders bevorzugt bei -20 bis -10°C durchgeführt.

Der durch die isotherme Absorption g vorgereinigte Chlorwasserstoffstrom wird intern der adiabaten Absorptionsstufe h zugeführt und dort weiter von Phosgen befreit. Zur Absorption wird im Gegenstrom Lösungsmittel 13, das durch den Wärmetauscher k mit Kaltsole auf Temperaturen von bevorzugt -50 bis +0°C, besonders bevorzugt -40 bis -20°C abgekühlt wurde, als Strom 14 am Kopf der adiabaten Absorption h aufgegeben. Der von Phosgen in der adiabaten Absorption h und von Lösungsmittel in der Nachkondensation i abgereinigte Chlorwasserstoffstrom 15 tritt am Kopf der Absorptionskolonne 51 aus. Die Kühlung der Nachkondensation erfolgt mit Kaltsole von bevorzugt -50 bis +0°C, besonders bevorzugt -40 bis -20°C. Zur Energierückgewinnung wird der Lösungsmittelstrom 13 im Wärmetauscher j vorgekühlt und der Chlorwasserstoffstrom 15 aufgewärmt.

Am Sumpf f der Phosgenabsorptionskolonne wird eine konzentrierte Phosgenlösung 11 abgezogen und zurück in die Reaktionsstufe gefördert.

Figur 3 zeigt eine schematische Darstellung der Auftrennung des Stroms enthaltend Chlorwasserstoff, Phosgen, Lösungsmittel sowie Leichtsieder und Inerte in einer Kombination von einer ein- oder mehrstufigen partiellen Kondensation (Schritt b)), gefolgt von einer Strippung des erhaltenen Kondensates (Schritt c)) und nochmaliger ein- oder mehrstufiger partieller Kondensation bei entsprechend tieferer Temperatur als in der partiellen Kondensation vor der Strippkolonne und weiterer Auftrennung des Brüdenstromes in einer Absorptionskolonne (Schritt e)) sowie Strippung der Phosgenlösung.

In der in Figur 3 gezeigten Ausführungsform kann die Reinheit des flüssigen Phosgenstroms 11 (Phosgenlösung), der am Sumpf der Absorptionskolonne 51 abgezogen wird, besonders bezüglich der Leichtsieder, die in Phosgen bzw. in dem Lösungsmittel löslich sind wie zum Bespiel Chlorwasserstoff, nochmals erhöht werden.

Der gasförmige Strom 1 enthaltend Chlorwasserstoff, Phosgen, Lösungsmittel sowie Leichtsieder und Inerte, der üblicherweise bei der Umsetzung von aliphatischen oder aromatischen Aminen mit Phosgen zu den entsprechenden Isocyanaten anfällt, wird in einer bevorzugten Ausführungsform der Erfindung zur Auftrennung zunächst in eine ein- oder mehrstufige partielle Kondensation a geleitet. Zusätzlich können dort oder in der partiellen Kondensationsstufe e nach der Strippkolonne phosgenhaltige Lösungsmittelströme 5 aus dem Isocyanatprozess aufgegeben werden, wie sie üblicherweise als Waschflüssigkeiten aus der Phosgenherstellung, Ringflüssigkeiten aus dem Vakuumsystem, Kondensate aus der Lösungsmitteldestillation, etc. anfallen können. Als Wärmetauscher eignen sich alle dem Stand der Technik entsprechenden Kondensatoren. Der Kondensationsschritt erfolgt üblicherweise mit luftgekühlter Kühlsole, bei bevorzugt 25 bis 50°C, besonders bevorzugt bei 30 bis 40°C. Das dabei enstehende Kondensat 2 wird als Zulauf auf den Kopf einer Strippkolonne 50 gegeben. Der Zulauf wird im strukturierten Packungsteil b der Kolonne in einen Kopfbrüdenstrom 3, der weitestgehend von Lösungsmittel abgereichert ist und wieder in die erste partielle Kondensationsstufe a geleitet wird, und in einem von Phosgen weitestgehend befreiten Lösungsmittelstrom 4 am Sumpf c der Kolonne aufgetrennt. Die dazu notwendigen Brüden werden aus der aus der Packung b ablaufenden Flüssigkeit mit dem Sumpfverdampfer d erzeugt.

Der Brüdenstrom 6 aus der ersten partiellen Kondensationsstufe a wird über eine zweite bei tieferer Temperatur betriebene ein- oder mehrstufige partielle Kondensation e geleitet. Als Wärmetauscher eignen sich alle dem Stand der Technik entsprechenden Kondensatoren. Der Kondensationsschritt wird bevorzugt mit Kaltsole bei bevorzugt -40 bis +10°C, besonders bevorzugt bei -20 bis -10°C durchgeführt. Die resultierenden Brüden- 7 und Kondensat-Ströme 8 werden in eine Phosgenabsorptionskolonne 51, einer Kombination aus isothermer g und adiabater Absorption h mit Nachkondensation i des Lösungsmittels und Energierückgewinnung j aus dem nur noch schwach Phosgen bzw. Lösungsmittelhaltigen Chlorwasserstoffstrom, zur weiteren Auftrennung geleitet. Zusätzlich werden kondensiertes Phosgen 9 und ein nichtkondensierter Phosgenstrom 10 der auch noch für Phosgen typische Inertgase enthält aus der Phosgenherstellung in den Sumpf f der Absorptionskolonne 51 gegeben. Der Zulauf 12 am Kopf der isothermen Absorption g besteht aus Lösungsmittel das ggf. auch durch Lösungsmittelströme die mit geringen Mengen Phosgen verunreinigt sind (wie z.B. Waschflüssigkeiten aus der Phosgenherstellung, Ringflüssigkeiten aus dem Vakuumsystem, Kondensate aus der Lösungsmitteldestillation, etc.) ersetzt werden kann. Die Kühlung der isothermen Absorption wird bevorzugt mit Kaltsole bei bevorzugt -40 bis +0°C, besonders bevorzugt bei -20 bis -10°C durchgeführt.

Der durch die isotherme Absorption g vorgereinigte Chlorwasserstoffstrom wird intern der adiabaten Absorptionsstufe h zugeführt und dort weiter von Phosgen befreit. Zur Absorption wird im Gegenstrom Lösungsmittel 13, das durch den Wärmetauscher k mit Kaltsole auf Temperaturen von bevorzugt -50 bis +0°C, besonders bevorzugt -40 bis -20°C abgekühlt wurde, als Strom 14 am Kopf der adiabaten Absorption h aufgegeben. Der von Phosgen in der adiabaten Absorption h und von Lösungsmittel in der Nachkondensation (i) abgereinigte Chlorwasserstoffstrom 15 tritt am Kopf der Absorptionskolonne 51 aus. Die Kühlung der Nachkondensation erfolgt mit Kaltsole von bevorzugt -50 bis +0°C, besonders bevorzugt -40 bis -20°C. Zur Energierückgewinnung wird der Lösungsmittelstrom 13 im Wärmetauscher j vorgekühlt und der Chlorwasserstoffstrom 15 aufgewärmt.

Die aus der partiellen Kondensation e und der isothermen Absorption g ablaufende und noch Leichtsieder enthaltende Phosgenlösung, wird im Packungsteil 1 durch den gasförmigen entgegenströmenden Phosgenstrom 10, der auch für die Phosgenherstellung typische Inertgase enthält, gestrippt.

Die Ausführung der Strippsektion 1 entspricht dem bekannten Stand der Technik. Die Auslegung erfolgt in Hinblick auf den gewünschten Leichtsiederanteil im Sumpfstrom, nach einer dem Fachmann bekannten Art und Weise wie sie für solche Trennungen üblich ist. Die apparative Ausführung kann eine Packungs-, Füllkörper- oder Bodenkolonnensektion sein.

Am Sumpf f der Phosgenabsorptionskolonne wird eine konzentrierte Phosgenlösung 11 abgezogen und zurück in die Reaktionsstufe gefördert.

In der in Figur 3 dargestellten Ausführungsform der vorliegenden Erfindung kann die Konzentration an Chlorwasserstoff und anderen Leichtsiedern in der Phosgenlösung (flüssiger Phosgenstrom) durch den unterhalb der isothermen Absorption g angeordneten Strippteil 1 noch weiter reduziert werden, ohne eine zusätzliche separate Kolonne mit zusätzlichen Wärmetauschern zu benötigen, indem ein phosgenhaltiger Brüdenstrom aus der Phosgenherstellung der Isocyanatsynthese (Strom enthaltend mindestens 50 Gew.-% an Phosgen bezogen auf das Gewicht des Stroms) als Strippgas eingesetzt und bei der Strippung kondensiert wird. Üblicherweise wird dieser Brüdenstrom in der Isocyanatsynthese in einer separaten Kondensation verflüssigt und anschließend der Phosgenlösung zugefügt. Im erfindungsgemäßen Verfahren erfolgt die Kondensation gegebenenfalls mittels der latenten Wärme der aus der isothermen Absorption ablaufenden Phosgenlösung.

### Beispiele:

a) Die Herstellung von Isocyanat und die dabei durchgeführte Trennung eines Stroms enthaltend Chlorwasserstoff, Phosgen, Lösungsmittel sowie Leichtsieder und Inerte der bei der Umsetzung von aliphatischen oder aromatischen Aminen mit Phosgen zu den entsprechenden Isocyanaten in einer dem Stand der Technik entsprechenden Reaktionsstufe entsteht, erfolgt nach dem erfindungsgemäßen Verfahren. Dabei erfolgt die Trennung durch eine zweistufige partielle Kondensation, wobei das Kondensat aus der ersten Stufe der partiellen Kondensation als Zulauf auf den Kopf einer Strippkolonne gegeben wird, das Sumpfprodukt der Strippkolonne zurück zum Frisch-Lösungsmitteltank geführt wird und die Kopfbrüden der Strippkolonne wieder zurück in die erste Stufe der partiellen Kondensation geleitet werden und die gesamten Brüden aus der ersten partiellen Kondensation in die zweite partielle Kondensation eingespeist werden, anschließende partielle isotherme Absorption des Phosgens mit Chlorbenzol im Gegenstrom, gefolgt von einer adiabaten Absorption des verbliebenen Phosgens mit Chlorbenzol im Gegenstrom und schließlich durch eine Nachkondensation des restlichen Chlorbenzols aus dem phosgen- und lösungsmittelarmen Chlorwasserstoffstrom, entsprechend der Figur 2.

Das kondensierte Phosgen und das größtenteils durch eine Waschkolonne in Chlorbenzol zurückgewonnene nichtkondensierbare Restphosgen aus der Phosgenherstellung werden ebenfalls in den Sumpf der Absorptionskolonne gegeben. Die dadurch am Sumpf erhaltene konzentrierte Phosgenlösung wird wieder zur Umsetzung mit dem Amin in die Reaktionsstufe zurückgeführt. Durch diese Kreislauffahrweise erhält man dabei wieder den zu trennenden Chlorwasserstoffstrom in seiner ursprünglichen Zusammensetzung und Menge.

Der gasförmige Chlorwasserstoffstrom 1 aus der Reaktionsstufe von 39,1 kg/h, wird in den Wärmetauscher der ersten partiellen Kondensationsstufe a, die bei 45°C (Kühlsole) betrieben wird eingespeist. Der Chlorwasserstoffstrom 1 enthält 23,8 Gew.-% Chlorwasserstoff, 49,3 Gew.-% Chlorbenzol, 26,9 Gew.-% Phosgen und geringe Mengen der für eine Isocyanatsynthese typischen Leichtsieder. Die Leichtsieder werden bei den nachfolgenden Zusammensetzungen der Stoffströme nicht mehr jedes Mal explizit genannt. Das dabei entstehende Kondensat 2 von 20,4 kg/h (0,7Gew.-% Chlorwasserstoff, 9,7 Gew.-% Phosgen, 89,6 Gew.-% Chlorbenzol) wird als Zulauf auf den Kopf einer Strippkolonne 50 gegeben. Der strukturierte Packungsteil b der Kolonne hat eine Höhe von 2m, die Kolonne einen Durchmesser von 55mm. Die Kopfbrüden 3 der Strippkolonne 50 von 2,3 kg/h (5,8 Gew.-% Chlorwasserstoff, 86,5 Gew.-% Phosgen, 7,7 Gew.-% Chlorbenzol) werden wieder in die erste partielle Kondensationsstufe a geleitet. Die von der Packung b ablaufende Flüssigkeit wird im Sumpf c der Strippkolonne 50 aufgefangen und teilweise mit dem Sumpfverdampfer d verdampft bzw. als gereinigter Chlorbenzolsumpfstrom 4 ausgeschleust. Dabei fallen 18,1 kg/h gereinigtes Chlorbenzol mit einem Restphosgengehalt von ca. 10ppm an.

Der Brüdenstrom 6 aus der ersten partiellen Kondensationsstufe a mit 21,0 kg/h (44,4 Gew.-% Chlorwasserstoff, 50,2 Gew.-% Phosgen, 5,4 Gew.-% Chlorbenzol) wird in die darauffolgende zweite partielle Kondensationsstufe e, die mit -17 °C (Kaltsole) betrieben wird geführt. Eine Zugabe von flüssigem phosgenhaltigem Chlorbenzol 5 aus dem Vakuumsystem wie in Figur 2 dargestellt wurde im Beispiel nicht durchgeführt.

Die nachfolgende Kombination aus isothermer und adiabater Absorption mit Nachkondensation des Lösungsmittels aus dem Abgas erfolgt in diesem Beispiel in einer Kombination aus mehreren Apparaten, die so verschaltet waren als ob die ausgeführten Schritte innerhalb eines Kolonnenkörpers angeordnet gewesen wären.

Der Brüdenstrom 7 mit 14,8 kg/h und -16,5 °C (61,55 Gew.-% Chlorwasserstoff, 38,4 Phosgen, 0,05 Gew.-% Chlorbenzol) und der Kondensatstrom 8 mit 6,2 kg/h und -16,5 °C (3,7 Gew.-% Chlorwasserstoff, 78,0 Gew.-% Phosgen, 18,3 Gew.-% Chlorbenzol) werden unterhalb der isothermen Absorptionsstufe g in den Sumpf f der Phosgenabsorptionskolonne zugespeist. Zusätzlich werden das kondensierte Phosgen 9 mit 11,2 kg/h (0,3 Gew.-% Chlorwasserstoff, 99,7 Gew.-% Phosgen) und das größtenteils durch eine Waschkolonne in Chlorbenzol zurückgewonnene nichtkondensierbare Restphosgen 10 mit 1,1 kg/h (0,4 Gew.-% Chlorwasserstoff, 19,4 Gew.-% Phosgen, 80,2 Gew.-% Chlorbenzol) aus der Phosgenherstellung in den Sumpf f der Absorptionskolonne 51 gegeben. Für die isotherme Absorption wurde ein stehender Wärmetauscher mit einem Rohr eingesetzt. Die Kühlung erfolgt mit Kühlsole bei -17 °C. Das Rohr hat einen Durchmesser von 54,4 mm, eine Länge von 3 m und ist mit Pall-Ringen mit 15x15 mm gefüllt. Der Zulauf 12 am Kopf der isothermen Absorption g besteht aus Chlorbenzol mit einer Menge von 3,7 kg/h und einer Temperatur von -38°C. Desweiteren gibt es den internen flüssigen Rückstrom aus der Packung der adiabaten Absorptionskolonne h auf den isothermen Absorptionsschritt. Der durch die isotherme Absorption g vorgereinigte Chlorwasserstoffstrom wird der adiabaten Absorptionsstufe h zugeführt. Die erhaltene Phosgenlösung 11 mit 43,2 kg/h und -6°C (2,1 Gew.-% Chlorwasserstoff, 50,8 Gew-% Phosgen, 47,1 Gew.-% Chlorbenzol) wird zurück in die Reaktionsstufe gefördert.

Die adiabate Absorption wird in einer Packungskolonne h mit dem Durchmesser von 55 mm durchgeführt. Die Packungshöhe beträgt 2m. Zur Absorption werden im Gegenstrom 14,9 kg/h Chlorbenzol 14, die durch den mit Kaltsole (-35 °C) betriebenen Wärmetauscher k auf -30 °C abgekühlt werden und der interne Kondensatstrom aus der Nachkondensation i, die mit -35 °C Kaltsole betrieben wird, am Kopf der adiabaten Absorption h aufgegeben. Der von Phosgen in der adiabaten Absorption h und Chlorbenzol in der Nachkondensation i abgereinigte am Kopf der Absorptionskolonne 51 austretende Chlorwasserstoffstrom 15 von 8,5 kg/h besteht aus 99,5 Gew.-% Chlorwasserstoff, 0,05 Gew.-% Chlorbenzol, 0,4 Gew.-% Phosgen und 0,05 Gew.-% der für eine Isocyanatsynthese typischen Leichtsieder. Der Kopfdruck beträgt 1,45 bar absolut, die Kopftemperatur -34°C. Eine rekuperative Wiederaufheizung des Chlorwasserstoffstromes 15 durch das zu kühlende Chlorbenzol 14 wie in Figur 2 dargestellt wurde im Beispiel nicht durchgeführt.
b) Zum Überprüfen der Wirksamkeit der Strippkolonne zur Erhöhung der Phosgenlösungskonzentration 11 bei gleichzeitig niedriger Phosgenverluste über den Chlorwasserstoffstrom 15 am Kopf der Absorptionskolonne 51 wird Beispiel a) in Kreislauffahrweise unter gleichen Randbedingungen allerdings ohne Betrieb der Strippkolonne durchgeführt. Das Kondensat 2 aus der ersten partiellen Kondensationsstufe a wird dementsprechend zusammen mit den Brüden 6 in die zweite partielle Kondensationsstufe e gegeben. Bei gleicher Aminlösungsmenge und Konzentration und gleichen Prozessparametern in der Reaktion und der Absorption und gleicher Restphosgenmenge im Chlorwasserstoffstrom 15 am Kopf der Absorptionskolonne 51 ergibt sich eine Phosgenlösung 11 mit lediglich 60,6 kg/h (2,6 Gew.-% Chlorwasserstoff, 61,2 Gew.-% Chlorbenzol, 36,2 Gew.-% Phosgen).

## Patentansprüche

1. Verfahren zur Herstellung von Isocyanaten, umfassend die folgenden Schritte:
a) Umsetzung von wenigstens einem Amin mit Phosgen in Gegenwart eines Lösungsmittels, wobei ein flüssiger Strom enthaltend das entsprechende Isocyanat und ggf. Lösungsmittel und ein gasförmiger Strom enthaltend Chlorwasserstoff, Phosgen, Lösungsmittel sowie Leichtsieder und Inerte erhalten wird,
b) Auftrennung des gasförmigen Stromes enthaltend Chlorwasserstoff, Phosgen, Lösungsmittel sowie Leichtsieder und Inerte durch partielle Kondensation, wobei eine flüssige Phase im Wesentlichen bestehend aus Lösungsmittel, Phosgen und Chlorwasserstoff erhalten und auf den Kopf einer Strippkolonne geführt wird, und wobei ein gasförmiger Strom im Wesentlichen enthaltend Chlorwasserstoff und Phosgen sowie Lösungsmittel in Konzentrationen von maximal 30 Gew.-%, bezogen auf das Gewicht des gasförmigen Stroms, sowie Leichtsieder und Inerte erhalten wird,
c) Auftrennung der in Schritt b) auf die Strippkolonne aufgegebenen flüssigen Phase im Wesentlichen bestehend aus Lösungsmittel, Phosgen und Chlorwasserstoff in eine Lösungsmittelphase, die dem Kolonnensumpf flüssig entnommen wird und die eine Phosgenkonzentration von < 0,1 Gew.-%, bezogen auf das Gewicht des flüssigen Sumpfstroms aufweist, sowie eine Gasphase, die am Kolonnenkopf entnommen wird und die Phosgen, Chlorwasserstoff, Leichtsieder und Inerte sowie Lösungsmittel in Konzentrationen von < 30 Gew.-%, bezogen auf das Gewicht der Gasphase enthält,
d) Rückführung der in Schritt c) erhaltenen Lösungsmittelphase in die Phosgenierung in Schritt a),
e) Einleitung der in Schritt c) erhaltenen Gasphase in eine Absorption, in der das in der Gasphase enthaltene Phosgen in dem gleichen Lösungsmittel absorbiert wird, das auch in der Phosgenierung in Schritt a) eingesetzt wird, wobei eine Phosgenlösung mit einer Konzentrationen von 20 - 80 Gew.-% Phosgen, bezogen auf das Gewicht der Phosgenlösung, erhalten wird,
f) Rückführung der erhaltenen Phosgenlösung in die Phosgenierung in Schritt a).

2. Verfahren nach Anspruch 1, bei dem die in Schritt e) erhaltene Phosgenlösung mit zusätslichem Phosgen vermischt wird und die so erhaltene konsentrierte Phosgenlösung in Schritt f) in die Phosgenierung in Schritt a) zurückgeführt wird.

3. Verfahren nach Anspruch 1 oder 2, bei dem in Schritt e) die Gasphase vor der Einleitung in die Absorption ein oder mehrmalig partiell kondensiert wird.

4. Verfahren nach einem der Anspruche 1 bis 3, weiterhin umfassend den folgenden Schritt:
g) Zusammenführung der in den Schritten b), c) und e) erhaltenen gasförmigen Ströme, wobei ein Chlorwasserstoff-Strom enthaltend Phosgen in Konzentrationen von maximal 0,5 Gew%, bevorzugt von maximal 0,2 Gew%, besonders bevorzugt von maximal 0,1 Gew%, bezogen auf das Gewicht des Chlorwasserstoff-Stroms erhalten wird.

5. Verfahren nach einem der Ansprüche 1 bis 4, bei dem die Phosgenierung in Schritt a) in der flüssigen Phase durchgeführt wird.

6. Verfahren nach Anspruch 5, bei dem das Amin zumindest teilweise zunächst mit der in Schritt d) zurückgeführten Lösungsmittelphase vermischt wird und die dabei erhaltene Aminlösung dann in die Umsetzung in Schritt a) eingeführt wird.

7. Verfahren nach Anspruch 5, bei dem das Phosgen zumindest teilweise zunächst mit der in Schritt d) zurückgeführten Lösungsmittelphase vermischt wird und die dabei erhaltene Phosgenlösung dann in die Umsetzung in Schritt a) eingeführt wird.

## Claims

1. Process for preparing isocyanates, which comprises the following steps:
a) reaction of at least one amine with phosgene in the presence of a solvent, to give a liquid stream containing the corresponding isocyanate and possibly solvent and a gaseous stream containing hydrogen chloride, phosgene, solvent and also low boilers and inerts,
b) fractionation of the gaseous stream containing hydrogen chloride, phosgene, solvent and also low boilers and inerts by partial condensation to give a liquid phase which consists essentially of solvent, phosgene and hydrogen chloride and is conveyed to the top of a stripping column and a gaseous stream consisting essentially of hydrogen chloride and phosgene and also solvent in concentrations of not more than 30% by weight, based on the weight of the gaseous stream, and also low boilers and inerts,
c) fractionation of the liquid phase which is introduced into the stripping column in step b) and consists essentially of solvent, phosgene and hydrogen chloride to give a solvent phase which is taken off in liquid form from the bottom of the column and has a phosgene concentration of < 0.1% by weight, based on the weight of the liquid bottom stream, and a gas phase which is taken off at the top of the column and contains phosgene, hydrogen chloride, low boilers and inerts and also solvent in concentrations of < 30% by weight, based on the weight of the gas phase,
d) recirculation of the solvent phase obtained in step c) to the phosgenation in step a),
e) introduction of the gas phase obtained in step c) into an absorption in which the phosgene present in the gas phase is absorbed in the same solvent which is also used in the phosgenation in step a), giving a phosgene solution having a concentration of 20-80% by weight of phosgene, based on the weight of the phosgene solution,
f) recirculation of the phosgene solution obtained to the phosgenation in step a).

2. Process according to Claim 1, wherein the phosgene solution obtained in step e) is mixed with additional phosgene and the concentrated phosgene solution obtained in this way is recirculated in step f) to the phosgenation in step a).

3. Process according to Claim 1 or 2, wherein the gas phase is partially condensed one or more times in step e) before introduction into the absorption.

4. Process according to any of Claims 1 to 3, which further comprises the following step:
g) combination of the gaseous streams obtained in steps b), c) and e) to give a hydrogen chloride stream containing phosgene in concentrations of not more than 0.5% by weight, preferably not more than 0.2% by weight, particularly preferably not more than 0.1% by weight, based on the weight of the hydrogen chloride stream.

5. Process according to any of Claims 1 to 4, wherein the phosgenation in step a) is carried out in the liquid phase.

6. Process according to Claim 5, wherein the amine is at least partly firstly mixed with the solvent phase recirculated in step d) and the resulting amine solution is then introduced into the reaction in step a).

7. Process according to Claim 5, wherein the phosgene is at least partly firstly mixed with the solvent phase recirculated in step d) and the resulting phosgene solution is then introduced into the reaction in step a).

## Revendications

1. Procédé pour la production d'isocyanates, comprenant les étapes suivantes :
a) transformation d'au moins une amine avec du phosgène en présence d'un solvant, un flux liquide, contenant l'isocyanate correspondant et le cas échéant un solvant, et un flux gazeux, contenant du chlorure d'hydrogène, du phosgène, du solvant ainsi que le cas échéant des substances à bas point d'ébullition et des substances inertes, étant obtenus,
b) séparation du flux gazeux, contenant du chlorure d'hydrogène, du phosgène, du solvant ainsi que des substances à bas point d'ébullition et des substances inertes, par condensation partielle, une phase liquide essentiellement constituée de solvant, de phosgène et de chlorure d'hydrogène étant obtenue et étant guidée sur la tête d'une colonne de rectification, et un flux gazeux contenant essentiellement du chlorure d'hydrogène et du phosgène ainsi que du solvant en des concentrations d'au maximum 30% en poids, par rapport au poids du flux gazeux, ainsi que des substances à bas point d'ébullition et des substances inertes étant obtenu,
c) séparation de la phase liquide introduite dans l'étape b) sur la colonne de rectification, essentiellement constituée de solvant, de phosgène et de chlorure d'hydrogène, en une phase de solvant, qui est prélevée sous forme liquide du fond de la colonne et qui présente une concentration en phosgène < 0,1% en poids par rapport au poids du flux liquide du fond, ainsi qu'en une phase gazeuse, qui est prélevée en tête de colonne et qui contient du phosgène, du chlorure d'hydrogène, des substances à bas point d'ébullition et des substances inertes ainsi que du solvant en des concentrations < 30% en poids, par rapport au poids de la phase gazeuse,
d) recyclage de la phase de solvant obtenue dans l'étape c) dans la phosgénation dans l'étape a),
e) introduction de la phase gazeuse obtenue dans l'étape c) dans une absorption, dans laquelle le phosgène contenu dans la phase gazeuse est absorbé dans le même solvant que celui qui est également utilisé dans la phosgénation dans l'étape a), une solution de phosgène présentant une concentration de 20-80% en poids de phosgène, par rapport au poids de la solution de phosgène, étant obtenue,
f) recyclage de la solution de phosgène obtenue dans la phosgénation dans l'étape a).

2. Procédé selon la revendication 1, dans lequel la solution de phosgène obtenue dans l'étape e) est mélangée avec du phosgène supplémentaire et la solution de phosgène concentrée ainsi obtenue dans l'étape f) est recyclée dans la phosgénation dans l'étape a).

3. Procédé selon la revendication 1 ou 2, dans lequel, dans l'étape e), la phase gazeuse est partiellement condensée, une fois ou plusieurs fois, avant l'introduction dans l'absorption.

4. Procédé selon l'une quelconque des revendications 1 à 3, comprenant en outre l'étape suivante :
g) réunion des flux gazeux obtenus dans les étapes b), c) et e), un flux de chlorure d'hydrogène contenant du phosgène en des concentrations d'au maximum 0,5% en poids, de préférence d'au maximum 0,2% en poids, de manière particulièrement préférée d'au maximum 0,1% en poids, par rapport au poids du flux de chlorure d'hydrogène, étant obtenu.

5. Procédé selon l'une quelconque des revendications 1 à 4, dans lequel la phosgénation dans l'étape a) est réalisée dans la phase liquide.

6. Procédé selon la revendication 5, dans lequel l'amine est, au moins partiellement, d'abord mélangée avec la phase de solvant recyclée dans l'étape d) et la solution d'amine ainsi obtenue est ensuite introduite dans la transformation dans l'étape a).

7. Procédé selon la revendication 5, dans lequel le phosgène est, au moins partiellement, d'abord mélangé avec la phase de solvant recyclée dans l'étape d) et la solution de phosgène ainsi obtenue est ensuite introduite dans la transformation dans l'étape a).
